# EUROPEAN PATENT APPLICATION

(11) **EP 2 119 773 A1**
(43) Date of publication of application: **18.11.2009**
(21) Application number: 09154132.6
(22) Date of filing: 26.06.2001
(51) Int. Cl.: C12N 9/20, C07K 14/37, A21D 8/04, A21D 10/00, C11D 3/386

(54) **Lipolytic enzymes from strains of fusarium and acremonium**

(30) Priority: 26.06.2000 DK 200000989; 26.06.2000 DK 200000990; 26.06.2000 DK 200000991; 04.08.2000 DK 200001172; 31.10.2000 US 703416; 31.10.2000 US 703414; 25.05.2001 DK 200100843
(62) Divisional of application: 01944983.4
(71) Applicant: Novozymes A/S, 2880 Bagsværd (DK); Novozymes Biotech, Inc., Davis, CA 95616 (US)
(72) Inventor: Tsutsumi, Noriko, Ishikawa Chiba 272 (JP); Sasaki, Yukiko, Ishikawa Chiba 273-0035 (JP); Rey, Michael W., Davis, CA 95616 (US); Zaretsky, Elizabeth, Davis, CA 95616 (US); Spendler, Tina, 2730 Herlev (DK); Vind, Jesper, Vaerloese 3500 (DK)
(74) Representative: Knudsen, Sten Lottrup

(57) **Abstract**

A group of genes encoding lipolytic enzymes with high homology have been isolated from strains of *Fusarium* and *Acremonium.*

SEQUENCE LISTING

<110> Novozymes

<120> Lipolytic enzymes and genes encoding them

<130> 10055.215-EP

<160> 60

<170> PatentIn version 3.0

<210> 1
<211> 2940
<212> DNA
<213> Fusarium venenatum

<220>
<221> CDS
<222> (1376)..(1465)

<220>
<221> CDS
<222> (1515)..(1681)

<220>
<221> CDS
<222> (1740)..(2529)

<220>
<221> mat_peptide
<222> (1515)..()

<400> 1
   aattcatgtg aatctactat gtaacagtat gttgtattgc attacccatc aacgttgaat 60
   cgttgcgacg taacggcccg gttcaagcga gatgtagata tgttggtagt taattgatgg 120
   gttaggtatt cttttcatca actcggtatt ctcattcccc agatatcggc acttgtcttt 180
   actccagatt tcatatcgca tcgagttata tacagtccca attgagtcga ctaccccgtc 240
   caaaacaggt tttctcacaa accaaccgca gcctaacaaa aagtcccttg tctttctgca 300
   ataaatgctg acaccccctg gctttttagg actgacggct cacgatgcag ccgttgcgat 360
   aattaattga caattacccg cacattgatg catacttggc ggtcaggtca ggtcaggctg 420
   aagcatacct attgggtcat ttatttgccg atcgtggtga aaagaatgca agtgataact 480
   agttacgagt cgctttatga aagatggttg gtcgaaactg tcaatatggc atgggcggca 540
   aatcgtttgg tctcaactct atagcatgta ctataattgg tcttttcatc acagtcacgc 600
   caaagtgcca gtctcagact atggaccaac cactttcctc cttcacgtct aaattgactt 660
   gatcaccaga ctcgaatatt ttttcttttc ttctataccc ctaggatcat acaatacgaa 720
   ccccaactca actcgagaga gagagtcccc ttcccaacat tttgacagcc cttgctcttc 780
   tcctcccagg atgtaacaga agctgaaagg gtacccctgt agcccacctt tacccaccat 840
   cttttccatc tgtatcggtg catcccatca caaccctcac gtggtccgag atcgtcgtta 900
   cccgtattgg aagctcactc cgggcccaac gagagattgg accaaggaaa aataactttg 960
   agacctcttc aagcagtcgg tcattcgtta ctgggatgtg tagtcgataa tgcggggtga 1020
   caggccctca atccagcacc caccatcatg ggcactgact gtactaccgg agcccatcat 1080
   ttcgtttttg ggtcctggcg tctacttgac cgactgagtt tgccaagatg gatggcatga 1140
   gagacagtgg ttaggctggg cgggtattgt gatgagagaa agcgagagac tagttagaag 1200
   caaagaaaaa agatatataa gctgtcacat ccctcatgaa catgctgttc ttgtaagtcg 1260
   ggatatcagg gccagcttca gtattcagta tcctttctga gggagttgca ccttgtcaca 1320
   gcttgtctgt ctatcactta tacttaccct tggaccacgt tctttgtctg tcaag atg 1378
   Met
   -30
   cat ctc cta tca ctc ctc tca att gcc acc ctt gcg gta gcc agc cct 1426
   His Leu Leu Ser Leu Leu Ser Ile Ala Thr Leu Ala Val Ala Ser Pro
   -25 -20 -15
   ctg agc gtt gaa gat tac gcc aag gct ctc gat gaa aga ggtaaaacga 1475
   Leu Ser Val Glu Asp Tyr Ala Lys Ala Leu Asp Glu Arg
   -10 -5 -1
   ttctctgttc ccataacaat tccaatactc acagaccta gct gtt tct gtc tct 1529
   Ala Val Ser Val Ser
   1 5
   acc aac gac ttt ggc aac ttc aag ttc tac atc cag cac ggt gcc gca 1577
   Thr Asn Asp Phe Gly Asn Phe Lys Phe Tyr Ile Gln His Gly Ala Ala
   10 15 20
   gca tac tgt aac tct gaa gcc gca gcc ggt gca aag gtc acc tgc gga 1625
   Ala Tyr Cys Asn Ser Glu Ala Ala Ala Gly Ala Lys Val Thr Cys Gly
   25 30 35
   gga aac ggt tgc cca acg gtc cag tcc aat ggt gcc acc atc gtg gca 1673
   Gly Asn Gly Cys Pro Thr Val Gln Ser Asn Gly Ala Thr Ile Val Ala
   40 45 50
   tct ttc ct  gtaagtctaa catatcacaa acacatcatc aactccaaac          1721
   Ser Phe Leu
       55
   ttacaaatct ctttatag t ggc tca aag act ggc atc ggt ggc tac gtc gcg   1773
                         Gly Ser Lys Thr Gly Ile Gly Gly Tyr Val Ala
                                     60                  65
   acc gac tct gca cgc aag gaa atc gtc ctc tcg gtt cgc ggt agc acc   1821
   Thr Asp Ser Ala Arg Lys Glu Ile Val Leu Ser Val Arg Gly Ser Thr
           70                  75                  80
   aac att cgc aac tgg ctt acc aac ctc gac ttc gac cag gat gac tgc   1869
   Asn Ile Arg Asn Trp Leu Thr Asn Leu Asp Phe Asp Gln Asp Asp Cys
       85                  90                  95
   agc ttg acc tcc ggc tgt gga gtg cac gga ggc ttc cag aga gcc tgg   1917
   Ser Leu Thr Ser Gly Cys Gly Val His Gly Gly Phe Gln Arg Ala Trp
   100                 105                 110                 115
   aat gag atc tcg gcc gca gca acc gcc gct gtc gca aag gcc cgc aag   1965
   Asn Glu Ile Ser Ala Ala Ala Thr Ala Ala Val Ala Lys Ala Arg Lys
                   120                 125                 130
   gcg aac cct tcg ttc aag gtc gtt gcc aca ggt cac tcg ttg ggt ggt   2013
   Ala Asn Pro Ser Phe Lys Val Val Ala Thr Gly His Ser Leu Gly Gly
               135                 140                 145
   gct gta gct aca cta gca ggt gcg aac ctg cga gtt ggt ggt acg cca   2061
   Ala Val Ala Thr Leu Ala Gly Ala Asn Leu Arg Val Gly Gly Thr Pro
           150                 155                 160
   gtt gac atc tac acc tac ggc tca ccc cga gtt gga aac acg caa ctc   2109
   Val Asp Ile Tyr Thr Tyr Gly Ser Pro Arg Val Gly Asn Thr Gln Leu
       165                 170                 175
   gct gcc ttc atc tct aac cag gct ggt gga gag ttc cgc gtt acg aac   2157
   Ala Ala Phe Ile Ser Asn Gln Ala Gly Gly Glu Phe Arg Val Thr Asn
   180                 185                 190                 195
   gcc aag gac ccc gtg cct cgt ctc ccc cct ctg gtc ttt gga tac cgg   2205
   Ala Lys Asp Pro Val Pro Arg Leu Pro Pro Leu Val Phe Gly Tyr Arg
                   200                 205                 210
   cac aca tcc ccc gag tac tgg ttg tct ggt agc gga ggt aac aag gtt   2253
   His Thr Ser Pro Glu Tyr Trp Leu Ser Gly Ser Gly Gly Asn Lys Val
               215                 220                 225
   gac tac acc atc aat gat gtc aag gtg tgt gag ggt gct gcc aac ctt   2301
   Asp Tyr Thr Ile Asn Asp Val Lys Val Cys Glu Gly Ala Ala Asn Leu
           230                 235                 240
   cag tgc aac ggt gga aca ctc gga ttg gat atc gac gcc cat ctc cac   2349
   Gln Cys Asn Gly Gly Thr Leu Gly Leu Asp Ile Asp Ala His Leu His
       245                 250                 255
   tac ttc cag gag acc gat gct tgc tct ggt tcc ggt atc gcg tgg aga   2397
   Tyr Phe Gln Glu Thr Asp Ala Cys Ser Gly Ser Gly Ile Ala Trp Arg
   260                 265                 270                 275
   aga tac agg agt gct aag cgt gag agc atc tcg gag agg gcc act atg   2445
   Arg Tyr Arg Ser Ala Lys Arg Glu Ser Ile Ser Glu Arg Ala Thr Met
                   280                 285                 290
   aca gat gcc gag ctg gag aag aag ctt aac aac tat gtt gcg atg gat   2493
   Thr Asp Ala Glu Leu Glu Lys Lys Leu Asn Asn Tyr Val Ala Met Asp
               295                 300                 305
   aag gag tat gtc aag act cac gcc aac cgc tca tcg tagtatgaca 2539
   Lys Glu Tyr Val Lys Thr His Ala Asn Arg Ser Ser
   310 315
   tttacgcagt aacgatataa ttaccataac aaaaactctg gataccattc tggtgcaagc 2599
   atggcgaaga aaacatcatt atctatgtga atgtatcata accatcctta cgccatgccg 2659
   ttgatcttac tactgagaca aaatactcag tcatgtacaa caaactccaa agcaccgaat 2719
   gacttctggc tttttggcaa agcacgaaac caatcattca aacccctcca cgaccatgcc 2779
   ctgcgcattg ggaacaccca cgagaatgac accacgaggc acgcggacac tcttcacctt 2839
   catgcaccca aagacattga cttcccggat attagggcat gctcggaaaa tggaacccag 2899
   aacaaaatcc gtcactgcct cacagaaact gatctccaat t 2940

<210> 2

<211> 349

<212> PRT

<213> Fusarium venenatum

<400> 2

Met His Leu Leu Ser Leu Leu Ser Ile Ala Thr Leu Ala Val Ala Ser
-30 -25 -20 -15

Pro Leu Ser Val Glu Asp Tyr Ala Lys Ala Leu Asp Glu Arg Ala Val
-10 -5 -1 1

Ser Val Ser Thr Asn Asp Phe Gly Asn Phe Lys Phe Tyr Ile Gln His
                5                   10                  15

Gly Ala Ala Ala Tyr Cys Asn Ser Glu Ala Ala Ala Gly Ala Lys Val
            20                  25                  30

Thr Cys Gly Gly Asn Gly Cys Pro Thr Val Gln Ser Asn Gly Ala Thr
35 40 45 50

Ile Val Ala Ser Phe Leu Gly Ser Lys Thr Gly Ile Gly Gly Tyr Val
55 60 65

Ala Thr Asp Ser Ala Arg Lys Glu Ile Val Leu Ser Val Arg Gly Ser
70 75 80

Thr Asn Ile Arg Asn Trp Leu Thr Asn Leu Asp Phe Asp Gln Asp Asp
                85                  90                  95

Cys Ser Leu Thr Ser Gly Cys Gly Val His Gly Gly Phe Gln Arg Ala
            100                 105                 110

Trp Asn Glu Ile Ser Ala Ala Ala Thr Ala Ala Val Ala Lys Ala Arg
115 120 125 130

Lys Ala Asn Pro Ser Phe Lys Val Val Ala Thr Gly His Ser Leu Gly
                135                 140                 145

Gly Ala Val Ala Thr Leu Ala Gly Ala Asn Leu Arg Val Gly Gly Thr
            150                 155                 160

Pro Val Asp Ile Tyr Thr Tyr Gly Ser Pro Arg Val Gly Asn Thr Gln
        165                 170                 175

Leu Ala Ala Phe Ile Ser Asn Gln Ala Gly Gly Glu Phe Arg Val Thr
    180                 185                 190

Asn Ala Lys Asp Pro Val Pro Arg Leu Pro Pro Leu Val Phe Gly Tyr
195                 200                 205                 210

Arg His Thr Ser Pro Glu Tyr Trp Leu Ser Gly Ser Gly Gly Asn Lys
                215                 220                 225

Val Asp Tyr Thr Ile Asn Asp Val Lys Val Cys Glu Gly Ala Ala Asn
            230                 235                 240

Leu Gln Cys Asn Gly Gly Thr Leu Gly Leu Asp Ile Asp Ala His Leu
        245                 250                 255

His Tyr Phe Gln Glu Thr Asp Ala Cys Ser Gly Ser Gly Ile Ala Trp
    260                 265                 270

Arg Arg Tyr Arg Ser Ala Lys Arg Glu Ser Ile Ser Glu Arg Ala Thr
275                 280                 285                 290

Met Thr Asp Ala Glu Leu Glu Lys Lys Leu Asn Asn Tyr Val Ala Met
                295                 300                 305

Asp Lys Glu Tyr Val Lys Thr His Ala Asn Arg Ser Ser
            310                 315

<210>  3

<211>  1161

<212>  DNA

<213>  Fusarium sulphureum


<220>

<221>  CDS

<222>  (1)..(94)


<220>

<221>  CDS
<222>  (147)..(312)

<220>
<221>  CDS
<222>  (369)..(1158)

<220>
<221>  mat_peptide
<222>  (94)..()

<400>  3
atg ctc ctc cta cca ctc ctc tca gcc gtc act ctc gcg gta gca agt        48
Met Leu Leu Leu Pro Leu Leu Ser Ala Val Thr Leu Ala Val Ala Ser
    -30                 -25                 -20

cca cta gct tcc gtc gaa gag tac gcc aag tct ctc gaa gac aga g          94
Pro Leu Ala Ser Val Glu Glu Tyr Ala Lys Ser Leu Glu Asp Arg
-15                 -10                 -5                  -1

gtaagcacca aactctcctc catatcatgc tatatactca tcacactccc ag ct  gtg     151
                                                          Ala Val
                                                          1

act gtg tct tcg tca gac tac aac aac ttc aag ttc tac atc caa cat       199
Thr Val Ser Ser Ser Asp Tyr Asn Asn Phe Lys Phe Tyr Ile Gln His
        5                   10                  15

ggc gcc gca gca tac tgt aac tcc gaa gcc tca gct ggc gca aag atc       247
Gly Ala Ala Ala Tyr Cys Asn Ser Glu Ala Ser Ala Gly Ala Lys Ile
    20                  25                  30

acc tgc gca agc aac ggt tgt cca acc gtc cag tcc aac ggc gca acc       295
Thr Cys Ala Ser Asn Gly Cys Pro Thr Val Gln Ser Asn Gly Ala Thr
35                  40                  45                  50

atc gtg gca tcc ttc ct  gtaagtcacg cccagtcaca aacatctcat              342
Ile Val Ala Ser Phe Leu
                55

acctcatact tatatgactt cttcag t ggt tcc aag act ggc atc ggc ggt        393
                               Gly Ser Lys Thr Gly Ile Gly Gly
                                           60

tat gtc gca aca gat tca tcc cgc aag gaa atc gtc gtc tcg atc cgt       441
Tyr Val Ala Thr Asp Ser Ser Arg Lys Glu Ile Val Val Ser Ile Arg
65                  70                  75                  80

gga agc agc aac atc cgc aac tgg ctt aca aac ctc gac ttt gac cag       489
Gly Ser Ser Asn Ile Arg Asn Trp Leu Thr Asn Leu Asp Phe Asp Gln
                85                  90                  95

tcc gac tgc agc ttg acc tcc ggc tgc ggc gta cac tcg ggc ttc cag       537
Ser Asp Cys Ser Leu Thr Ser Gly Cys Gly Val His Ser Gly Phe Gln
            100                 105                 110

aac gcc tgg gac gag atc tcg gag aga gcc act gct gct gtg gcc aag       585

Asn Ala Trp Asp Glu Ile Ser Glu Arg Ala Thr Ala Ala Val Ala Lys
        115                 120                 125

gca cgc aag gca aac tct ggt ttc aag gtc att gct aca ggc cac tcc      633
Ala Arg Lys Ala Asn Ser Gly Phe Lys Val Ile Ala Thr Gly His Ser
    130                 135                 140

ctc ggt ggt gcg gtc gct aca ttg gct gct gcg aat ctg agg gtt ggt      681
Leu Gly Gly Ala Val Ala Thr Leu Ala Ala Ala Asn Leu Arg Val Gly
145                 150                 155                 160

ggc aca ccc gtg gac atc tac acg tac ggt gct cct cga gtg ggc aac      729
Gly Thr Pro Val Asp Ile Tyr Thr Tyr Gly Ala Pro Arg Val Gly Asn
                165                 170                 175

gcc cag ctt tca gcg ttc atc tcg aac caa gct ggc ggg gaa tat cgc      777
Ala Gln Leu Ser Ala Phe Ile Ser Asn Gln Ala Gly Gly Glu Tyr Arg
            180                 185                 190

gtt act cac gcc aga gac ccc gtg cct cgt ctg ccc cct ctg gtg ttt      825
Val Thr His Ala Arg Asp Pro Val Pro Arg Leu Pro Pro Leu Val Phe
        195                 200                 205

gga tac agg cac act tcg ccc gag tac tgg cta tct ggc ggc ggt ggc      873
Gly Tyr Arg His Thr Ser Pro Glu Tyr Trp Leu Ser Gly Gly Gly Gly
    210                 215                 220

gac aag att gat tat acc atc agc gat atc aag gtc tgt gag ggc gcc      921
Asp Lys Ile Asp Tyr Thr Ile Ser Asp Ile Lys Val Cys Glu Gly Ala
225                 230                 235                 240

gct aat ctc cag tgt aac ggt ggc acg ctg ggt ttg gac att gcg gct      969
Ala Asn Leu Gln Cys Asn Gly Gly Thr Leu Gly Leu Asp Ile Ala Ala
                245                 250                 255

cat ctg cat tac ttc cag cac act gat gct tgc tcg gcg gga ggc att     1017
His Leu His Tyr Phe Gln His Thr Asp Ala Cys Ser Ala Gly Gly Ile
            260                 265                 270

tct ttt aga cga tac agg agt gct aag cgt gaa ggt atc gcc aag agg     1065
Ser Phe Arg Arg Tyr Arg Ser Ala Lys Arg Glu Gly Ile Ala Lys Arg
        275                 280                 285

gct gat atg tcg gat gct gag ctg gag aag aag ctc aac tct tat gtt     1113
Ala Asp Met Ser Asp Ala Glu Leu Glu Lys Lys Leu Asn Ser Tyr Val
    290                 295                 300

gag atg gat aag gag tat gtg gat agc cat aag aat cgt tca tca taa     1161
Glu Met Asp Lys Glu Tyr Val Asp Ser His Lys Asn Arg Ser Ser
305                 310                 315

<210>  4

<211>  350

<212>  PRT

<213>  Fusarium sulphureum

<400>  4

Met Leu Leu Leu Pro Leu Leu Ser Ala Val Thr Leu Ala Val Ala Ser
    -30                 -25                 -20

Pro Leu Ala Ser Val Glu Glu Tyr Ala Lys Ser Leu Glu Asp Arg Ala

-15                -10                -5              -1  1

Val Thr Val Ser Ser Ser Asp Tyr Asn Asn Phe Lys Phe Tyr Ile Gln
            5                   10                  15

His Gly Ala Ala Ala Tyr Cys Asn Ser Glu Ala Ser Ala Gly Ala Lys
        20                  25                  30

Ile Thr Cys Ala Ser Asn Gly Cys Pro Thr Val Gln Ser Asn Gly Ala
    35                  40                  45

Thr Ile Val Ala Ser Phe Leu Gly Ser Lys Thr Gly Ile Gly Gly Tyr
50                  55                  60                  65

Val Ala Thr Asp Ser Ser Arg Lys Glu Ile Val Val Ser Ile Arg Gly
                70                  75                  80

Ser Ser Asn Ile Arg Asn Trp Leu Thr Asn Leu Asp Phe Asp Gln Ser
            85                  90                  95

Asp Cys Ser Leu Thr Ser Gly Cys Gly Val His Ser Gly Phe Gln Asn
        100                 105                 110

Ala Trp Asp Glu Ile Ser Glu Arg Ala Thr Ala Ala Val Ala Lys Ala
    115                 120                 125

Arg Lys Ala Asn Ser Gly Phe Lys Val Ile Ala Thr Gly His Ser Leu
130                 135                 140                 145

Gly Gly Ala Val Ala Thr Leu Ala Ala Ala Asn Leu Arg Val Gly Gly
                150                 155                 160

Thr Pro Val Asp Ile Tyr Thr Tyr Gly Ala Pro Arg Val Gly Asn Ala
            165                 170                 175

Gln Leu Ser Ala Phe Ile Ser Asn Gln Ala Gly Gly Glu Tyr Arg Val
        180                 185                 190

Thr His Ala Arg Asp Pro Val Pro Arg Leu Pro Pro Leu Val Phe Gly
    195                 200                 205

Tyr Arg His Thr Ser Pro Glu Tyr Trp Leu Ser Gly Gly Gly Gly Asp
210                 215                 220                 225

Lys Ile Asp Tyr Thr Ile Ser Asp Ile Lys Val Cys Glu Gly Ala Ala
                230                 235                 240

Asn Leu Gln Cys Asn Gly Gly Thr Leu Gly Leu Asp Ile Ala Ala His
            245                 250                 255

Leu His Tyr Phe Gln His Thr Asp Ala Cys Ser Ala Gly Gly Ile Ser
        260                 265                 270

Phe Arg Arg Tyr Arg Ser Ala Lys Arg Glu Gly Ile Ala Lys Arg Ala
    275                 280                 285

Asp Met Ser Asp Ala Glu Leu Glu Lys Lys Leu Asn Ser Tyr Val Glu
290                 295                 300                 305

Met Asp Lys Glu Tyr Val Asp Ser His Lys Asn Arg Ser Ser
                310                 315

<210>  5

<211>  1103

<212>  DNA

<213>  Acremonium berkeleyanum

<220>

<221>  CDS

<222>  (1)..(85)

<220>

<221>  CDS

<222>  (141)..(306)

<220>

<221>  CDS

<222>  (365)..(1100)

<220>

<221>  mat_peptide

<222>  (85)..()

<400>  5
atg ctc gcc cta tcc ctt ctt tct att gct gcc ctg gcg gta gct agt        48
Met Leu Ala Leu Ser Leu Leu Ser Ile Ala Ala Leu Ala Val Ala Ser
            -25                 -20                 -15

ccc ttg gcc gat tat tcc aag gcc ttg gaa gac cga g gtgagtccaa           95
Pro Leu Ala Asp Tyr Ser Lys Ala Leu Glu Asp Arg
        -10                 -5              -1

ccacttacac acctccaggc agaaacgatt gctaagtaaa cacag cc  atc tcg gtc     151
                                                  Ala Ile Ser Val
                                                  1

acc gat gga gac ctg aac aac ttc aag ttc tac gtc caa cac gct gcc     199
Thr Asp Gly Asp Leu Asn Asn Phe Lys Phe Tyr Val Gln His Ala Ala
5                   10                  15                  20

gcc gca tac tgc aat gtc aac act cca gcg ggt caa gct gtg aag tgc     247
Ala Ala Tyr Cys Asn Val Asn Thr Pro Ala Gly Gln Ala Val Lys Cys
                25                  30                  35

ggc gga tcc aca tgc tct gct gtc gaa ggc gac agg gtg aca gtc gtc     295
Gly Gly Ser Thr Cys Ser Ala Val Glu Gly Asp Arg Val Thr Val Val
            40                  45                  50

gca tcc ttc aa  gtacgttcct cttctatgtt agacttaaag aagcaatccc         346
Ala Ser Phe Asn
        55

agctaacgtc cccatcag c ggc gct gga aca gga att ggc ggc tat gta gcc   398
                      Gly Ala Gly Thr Gly Ile Gly Gly Tyr Val Ala
                                  60                  65

acc gac aac gcc cgc tct gag atc gtt gtc tcc atc cgc ggt agt agc     446
Thr Asp Asn Ala Arg Ser Glu Ile Val Val Ser Ile Arg Gly Ser Ser
        70                  75                  80

aat atc cgc aac tgg att gcc aac ata gag ttt gcg cag cag gac tgc     494
Asn Ile Arg Asn Trp Ile Ala Asn Ile Glu Phe Ala Gln Gln Asp Cys
    85                  90                  95

tcc ctt gtt gct ggc tgc ggt gtg cac act ggc ttc cag aag gca tgg     542
Ser Leu Val Ala Gly Cys Gly Val His Thr Gly Phe Gln Lys Ala Trp
100                 105                 110                 115

aac gag atc tcc gcc aac gtc aag gcc gct gtg gca tct gcg aag cag     590
Asn Glu Ile Ser Ala Asn Val Lys Ala Ala Val Ala Ser Ala Lys Gln
                120                 125                 130

gcg aac cca agc tac aag atc atc tcg act ggt cac tcc ctc ggc ggt     638
Ala Asn Pro Ser Tyr Lys Ile Ile Ser Thr Gly His Ser Leu Gly Gly
            135                 140                 145

gcg gtg gct acc ctc gcg gcc gcg tac ctg cgt aag gat ggc aat gct     686
Ala Val Ala Thr Leu Ala Ala Ala Tyr Leu Arg Lys Asp Gly Asn Ala
        150                 155                 160

gtc gat ctg tac aca tac ggc tcg cca cga gtc ggc aac ggt gtc ttc     734
Val Asp Leu Tyr Thr Tyr Gly Ser Pro Arg Val Gly Asn Gly Val Phe
    165                 170                 175

tct aac ttc gtc agc caa caa gcc ggc tct gaa ttc cgc gtc acc cac     782
Ser Asn Phe Val Ser Gln Gln Ala Gly Ser Glu Phe Arg Val Thr His
180                 185                 190                 195

ggc gac gac ccc gtc ccc cgt ctg ccc cca atc gtc ttc ggc tac cgc     830
Gly Asp Asp Pro Val Pro Arg Leu Pro Pro Ile Val Phe Gly Tyr Arg
                200                 205                 210

cac acc acc ccg gag tac tgg ctc gac ggc ggc tcc ctg gac gtg acg     878
His Thr Thr Pro Glu Tyr Trp Leu Asp Gly Gly Ser Leu Asp Val Thr
            215                 220                 225

tac aac ctc gac gag atc aag gtg tgc gag ggc aat gcg aat gtg aac     926
Tyr Asn Leu Asp Glu Ile Lys Val Cys Glu Gly Asn Ala Asn Val Asn
        230                 235                 240

tgc aac ggt ggt acg ttt ggc ctt gat atc ctt gcg cat ttg cgg tac     974
Cys Asn Gly Gly Thr Phe Gly Leu Asp Ile Leu Ala His Leu Arg Tyr
    245                 250                 255

cta cag gat gta tca ggt tgt gcg ccg atc ggc atc ttc tgg aag cgc     1022

Leu Gln Asp Val Ser Gly Cys Ala Pro Ile Gly Ile Phe Trp Lys Arg
260                 265                 270                 275

gag gag atg tcg gat gaa gag ttg gag aag aag gtg aat gag tat gtc      1070
Glu Glu Met Ser Asp Glu Glu Leu Glu Lys Lys Val Asn Glu Tyr Val
                280                 285                 290

cag gcc gac aag gac ttt gtg gct ggg ttg tag                          1103
Gln Ala Asp Lys Asp Phe Val Ala Gly Leu
            295                 300

<210>  6

<211>  329

<212>  PRT

<213>  Acremonium berkeleyanum

<400>  6

Met Leu Ala Leu Ser Leu Leu Ser Ile Ala Ala Leu Ala Val Ala Ser
            -25                 -20                 -15

Pro Leu Ala Asp Tyr Ser Lys Ala Leu Glu Asp Arg Ala Ile Ser Val
        -10                 -5                  -1  1

Thr Asp Gly Asp Leu Asn Asn Phe Lys Phe Tyr Val Gln His Ala Ala
5                   10                  15                  20

Ala Ala Tyr Cys Asn Val Asn Thr Pro Ala Gly Gln Ala Val Lys Cys
                25                  30                  35

Gly Gly Ser Thr Cys Ser Ala Val Glu Gly Asp Arg Val Thr Val Val
            40                  45                  50

Ala Ser Phe Asn Gly Ala Gly Thr Gly Ile Gly Gly Tyr Val Ala Thr
        55                  60                  65

Asp Asn Ala Arg Ser Glu Ile Val Val Ser Ile Arg Gly Ser Ser Asn
    70                  75                  80

Ile Arg Asn Trp Ile Ala Asn Ile Glu Phe Ala Gln Gln Asp Cys Ser
85                  90                  95                  100

Leu Val Ala Gly Cys Gly Val His Thr Gly Phe Gln Lys Ala Trp Asn
                105                 110                 115

Glu Ile Ser Ala Asn Val Lys Ala Ala Val Ala Ser Ala Lys Gln Ala
            120                 125                 130

Asn Pro Ser Tyr Lys Ile Ile Ser Thr Gly His Ser Leu Gly Gly Ala
        135                 140                 145

Val Ala Thr Leu Ala Ala Ala Tyr Leu Arg Lys Asp Gly Asn Ala Val

150                 155                 160

Asp Leu Tyr Thr Tyr Gly Ser Pro Arg Val Gly Asn Gly Val Phe Ser
165                 170                 175                 180

Asn Phe Val Ser Gln Gln Ala Gly Ser Glu Phe Arg Val Thr His Gly
                185                 190                 195

Asp Asp Pro Val Pro Arg Leu Pro Pro Ile Val Phe Gly Tyr Arg His
            200                 205                 210

Thr Thr Pro Glu Tyr Trp Leu Asp Gly Gly Ser Leu Asp Val Thr Tyr
        215                 220                 225

Asn Leu Asp Glu Ile Lys Val Cys Glu Gly Asn Ala Asn Val Asn Cys
    230                 235                 240

Asn Gly Gly Thr Phe Gly Leu Asp Ile Leu Ala His Leu Arg Tyr Leu
245                 250                 255                 260

Gln Asp Val Ser Gly Cys Ala Pro Ile Gly Ile Phe Trp Lys Arg Glu
                265                 270                 275

Glu Met Ser Asp Glu Glu Leu Glu Lys Lys Val Asn Glu Tyr Val Gln
            280                 285                 290

Ala Asp Lys Asp Phe Val Ala Gly Leu
        295                 300

<210>  7

<211>  1159

<212>  DNA

<213>  Fusarium culmorum


<220>

<221>  CDS

<222>  (1)..(91)


<220>

<221>  CDS

<222>  (144)..(309)


<220>

<221>  CDS

<222>  (367)..(1156)

<220>

<221>  mat_peptide

<222>  (91)..()

<400>  7
atg cgt ctc ctg tca ctc ctc tca gtt gtc acc ctt gtg gta gcc agc      48
Met Arg Leu Leu Ser Leu Leu Ser Val Val Thr Leu Val Val Ala Ser
-30                 -25                 -20                 -15

cct ctg agc gtt gaa gaa tac gcc aag gct ctc gat gaa cga g            91
Pro Leu Ser Val Glu Glu Tyr Ala Lys Ala Leu Asp Glu Arg
                -10                 -5                  -1

gtacacacga tcatcattcc tataacaaac tccatactca caaagtctct ag ct  gtc    148
                                                          Ala Val
                                                          1

tct gtc tcc acc acc gac ttt ggc aat ttc aag ttc tac atc cag cac      196
Ser Val Ser Thr Thr Asp Phe Gly Asn Phe Lys Phe Tyr Ile Gln His
        5                   10                  15

ggc gcc gca gca tac tgc aac tcc gaa gcc ccg gcc ggt gca aag gtc      244
Gly Ala Ala Ala Tyr Cys Asn Ser Glu Ala Pro Ala Gly Ala Lys Val
    20                  25                  30

acc tgc agc gga aac ggc tgt cca act gtt cag tcc aac ggt gtt acc      292
Thr Cys Ser Gly Asn Gly Cys Pro Thr Val Gln Ser Asn Gly Val Thr
35                  40                  45                  50

atc gtg gca tcc ttc ac  gtaagtccca cccggcacaa acacatcacc             339
Ile Val Ala Ser Phe Thr
                55

aactccaagc ttacacgttt ctctcag t gga tcc aag act gga atc ggc ggc      391
                                Gly Ser Lys Thr Gly Ile Gly Gly
                                            60

tac gtc gct aca gac cct aca cgc aag gag atc gtc gtc tcg ttc cgt      439
Tyr Val Ala Thr Asp Pro Thr Arg Lys Glu Ile Val Val Ser Phe Arg
65                  70                  75                  80

ggt agc atc aac atc cgc aac tgg ctt acc aac ctc gac ttc gac cag      487
Gly Ser Ile Asn Ile Arg Asn Trp Leu Thr Asn Leu Asp Phe Asp Gln
                85                  90                  95

gac gac tgc agc ctg acc tcg ggc tgt ggt gtt cac tca ggc ttc cag      535
Asp Asp Cys Ser Leu Thr Ser Gly Cys Gly Val His Ser Gly Phe Gln
            100                 105                 110

aaa gcc tgg aac gag atc tca gcc gcg gca acc gcc gct gtc gca aag      583
Lys Ala Trp Asn Glu Ile Ser Ala Ala Ala Thr Ala Ala Val Ala Lys
        115                 120                 125

gcc cgc aag gca aac cct tcg ttc aag gtc gtc tcc gta ggt cac tcc      631
Ala Arg Lys Ala Asn Pro Ser Phe Lys Val Val Ser Val Gly His Ser
    130                 135                 140

ctg ggt ggt gct gta gct aca ctg gca ggc gcg aac cta cga gtt ggt      679
Leu Gly Gly Ala Val Ala Thr Leu Ala Gly Ala Asn Leu Arg Val Gly
145                 150                 155                 160

gga aca ccc ctt gac att tac acc tac ggt tca ccc cga gtt gga aac        727
Gly Thr Pro Leu Asp Ile Tyr Thr Tyr Gly Ser Pro Arg Val Gly Asn
                165                 170                 175

aca cag ctc gct gct ttt gtc tcg aac cag gct ggt gga gag ttc cgc        775
Thr Gln Leu Ala Ala Phe Val Ser Asn Gln Ala Gly Gly Glu Phe Arg
            180                 185                 190

gtt acg aac gcc aaa gac ccc gtg cct cgt ctc ccc cct ctg atc ttt        823
Val Thr Asn Ala Lys Asp Pro Val Pro Arg Leu Pro Pro Leu Ile Phe
        195                 200                 205

gga tac cga cac aca tcc ccc gag tac tgg ctg tct ggc agc gga ggt        871
Gly Tyr Arg His Thr Ser Pro Glu Tyr Trp Leu Ser Gly Ser Gly Gly
    210                 215                 220

gac aag atc gac tac acc atc aac gat gtc aag gtc tgt gaa ggt gcc        919
Asp Lys Ile Asp Tyr Thr Ile Asn Asp Val Lys Val Cys Glu Gly Ala
225                 230                 235                 240

gcc aac ctc cag tgc aac ggt gga aca ctc gga ttg gat atc gat gcc        967
Ala Asn Leu Gln Cys Asn Gly Gly Thr Leu Gly Leu Asp Ile Asp Ala
                245                 250                 255

cat ctc cac tac ttc cag gca act gat gct tgc tct gct ggc ggc atc       1015
His Leu His Tyr Phe Gln Ala Thr Asp Ala Cys Ser Ala Gly Gly Ile
            260                 265                 270

tcg tgg aga aga tac agg agc gcc aag cgt gag agc atc tca gag agg       1063
Ser Trp Arg Arg Tyr Arg Ser Ala Lys Arg Glu Ser Ile Ser Glu Arg
        275                 280                 285

gct acc atg acc gac gcc gag ctc gag aag aag ctc aac agc tat gtt       1111
Ala Thr Met Thr Asp Ala Glu Leu Glu Lys Lys Leu Asn Ser Tyr Val
    290                 295                 300

gag atg gat aag gag tat atc aag act cac gcc agc cgc tca tca tag       1159
Glu Met Asp Lys Glu Tyr Ile Lys Thr His Ala Ser Arg Ser Ser
305                 310                 315

<210>  8

<211>  349

<212>  PRT

<213>  Fusarium culmorum

<400>  8

Met Arg Leu Leu Ser Leu Leu Ser Val Val Thr Leu Val Val Ala Ser
-30                 -25                 -20                 -15

Pro Leu Ser Val Glu Glu Tyr Ala Lys Ala Leu Asp Glu Arg Ala Val
                -10                 -5                  -1  1

Ser Val Ser Thr Thr Asp Phe Gly Asn Phe Lys Phe Tyr Ile Gln His
        5                   10                  15

Gly Ala Ala Ala Tyr Cys Asn Ser Glu Ala Pro Ala Gly Ala Lys Val
    20                  25                  30

Thr Cys Ser Gly Asn Gly Cys Pro Thr Val Gln Ser Asn Gly Val Thr
35                  40                  45                  50

Ile Val Ala Ser Phe Thr Gly Ser Lys Thr Gly Ile Gly Gly Tyr Val
                55                  60                  65

Ala Thr Asp Pro Thr Arg Lys Glu Ile Val Val Ser Phe Arg Gly Ser
            70                  75                  80

Ile Asn Ile Arg Asn Trp Leu Thr Asn Leu Asp Phe Asp Gln Asp Asp
        85                  90                  95

Cys Ser Leu Thr Ser Gly Cys Gly Val His Ser Gly Phe Gln Lys Ala
    100                 105                 110

Trp Asn Glu Ile Ser Ala Ala Ala Thr Ala Ala Val Ala Lys Ala Arg
115                 120                 125                 130

Lys Ala Asn Pro Ser Phe Lys Val Val Ser Val Gly His Ser Leu Gly
                135                 140                 145

Gly Ala Val Ala Thr Leu Ala Gly Ala Asn Leu Arg Val Gly Gly Thr
            150                 155                 160

Pro Leu Asp Ile Tyr Thr Tyr Gly Ser Pro Arg Val Gly Asn Thr Gln
        165                 170                 175

Leu Ala Ala Phe Val Ser Asn Gln Ala Gly Gly Glu Phe Arg Val Thr
    180                 185                 190

Asn Ala Lys Asp Pro Val Pro Arg Leu Pro Pro Leu Ile Phe Gly Tyr
195                 200                 205                 210

Arg His Thr Ser Pro Glu Tyr Trp Leu Ser Gly Ser Gly Gly Asp Lys
                215                 220                 225

Ile Asp Tyr Thr Ile Asn Asp Val Lys Val Cys Glu Gly Ala Ala Asn
            230                 235                 240

Leu Gln Cys Asn Gly Gly Thr Leu Gly Leu Asp Ile Asp Ala His Leu
        245                 250                 255

His Tyr Phe Gln Ala Thr Asp Ala Cys Ser Ala Gly Gly Ile Ser Trp
    260                 265                 270

Arg Arg Tyr Arg Ser Ala Lys Arg Glu Ser Ile Ser Glu Arg Ala Thr
275                 280                 285                 290

Met Thr Asp Ala Glu Leu Glu Lys Lys Leu Asn Ser Tyr Val Glu Met
                295                 300                 305

Asp Lys Glu Tyr Ile Lys Thr His Ala Ser Arg Ser Ser

310                 315

<210>  9

<211>  1150

<212>  DNA

<213>  Fusarium solani

<220>

<221>  CDS

<222>  (1)..(93)

<220>

<221>  CDS

<222>  (149)..(316)

<220>

<221>  CDS

<222>  (365)..(1147)

<220>

<221>  mat_peptide

<222>  (149)..()

<400>  9
atg cgc ctg ctt cct ctc ctc tcg gtc gta acg ctc act gcg gcg agt        48
Met Arg Leu Leu Pro Leu Leu Ser Val Val Thr Leu Thr Ala Ala Ser
    -30                 -25                 -20

cct ata gcc tcc gtc cag gag tac act gac gcc ttg gag aag aga            93
Pro Ile Ala Ser Val Gln Glu Tyr Thr Asp Ala Leu Glu Lys Arg
-15                 -10                 -5                  -1

ggtaaccacc aacactcccc taagacagac ctcgccctaa caagtaaact ctagt gct      151
                                                             Ala
                                                             1

atc acc gcc tct caa ctt gac tat gaa aac ttc aag ttt tac atc cag       199
Ile Thr Ala Ser Gln Leu Asp Tyr Glu Asn Phe Lys Phe Tyr Ile Gln
            5                   10                  15

cac ggt gcc gca gcg tat tgc aac tct gag acg gcc tct ggt caa aaa       247
His Gly Ala Ala Ala Tyr Cys Asn Ser Glu Thr Ala Ser Gly Gln Lys
        20                  25                  30

ata acc tgc aac gac aac ggc tgc aaa ggc atc gag gcc aac aac gcc       295
Ile Thr Cys Asn Asp Asn Gly Cys Lys Gly Ile Glu Ala Asn Asn Ala
    35                  40                  45

ata atc gta gca tcc ttc gtg taagctcccc tttcccctca cggaaccctt 346
Ile Ile Val Ala Ser Phe Val
50                  55

caactgacac gtagcagc ggc acg ggc act ggc atc gga ggc tac gtc tcc 397
Gly Thr Gly Thr Gly Ile Gly Gly Tyr Val Ser
            60                  65

act gac aat gtc cgt aag gag att gtc ctc tcg att cgc ggc agc agc 445
Thr Asp Asn Val Arg Lys Glu Ile Val Leu Ser Ile Arg Gly Ser Ser
        70                  75                  80

aac atc cgc aac tgg ctc acc aac gtc gac ttt ggc cag tcc agc tgc 493
Asn Ile Arg Asn Trp Leu Thr Asn Val Asp Phe Gly Gln Ser Ser Cys
    85                  90                  95

tcc tac gtc cgc gac tgc gga gtc cac acg ggc ttc cgc aat gcc tgg 541
Ser Tyr Val Arg Asp Cys Gly Val His Thr Gly Phe Arg Asn Ala Trp
100                 105                 110                 115

gat gag att gcc cag cgc gcg agg gac gcc gtt gcc aag gcc cgc gcc 589
Asp Glu Ile Ala Gln Arg Ala Arg Asp Ala Val Ala Lys Ala Arg Ala
                120                 125                 130

atg aac ccg tcc tac aag gtc atc tcc acg ggc cac tct ctc ggc ggt 637
Met Asn Pro Ser Tyr Lys Val Ile Ser Thr Gly His Ser Leu Gly Gly
            135                 140                 145

gct gtc gca act ctg ggt gcc gct gac ctg agg tcc aag gga acc gca 685
Ala Val Ala Thr Leu Gly Ala Ala Asp Leu Arg Ser Lys Gly Thr Ala
        150                 155                 160

gtt gac atc ttc acc ttt ggt gct ccc cgt gta ggc aac gct gaa ctc 733
Val Asp Ile Phe Thr Phe Gly Ala Pro Arg Val Gly Asn Ala Glu Leu
    165                 170                 175

tca gca ttc atc acg gcc cag gcc ggc ggc gag ttc cgt gtc act cat 781
Ser Ala Phe Ile Thr Ala Gln Ala Gly Gly Glu Phe Arg Val Thr His
180                 185                 190                 195

ggc cgt gat ccc gtg ccc cgt ctg cct ccc atc gtc ttt ggc tac aga 829
Gly Arg Asp Pro Val Pro Arg Leu Pro Pro Ile Val Phe Gly Tyr Arg
                200                 205                 210

cac aca tcg ccc gag tac tgg ctg gcc ggc ggt gca tcc acc aag atc 877
His Thr Ser Pro Glu Tyr Trp Leu Ala Gly Gly Ala Ser Thr Lys Ile
            215                 220                 225

gac tac tcc gtc aac gac atc aag gtc tgt gaa ggc gcc gcc aat ctc 925
Asp Tyr Ser Val Asn Asp Ile Lys Val Cys Glu Gly Ala Ala Asn Leu
        230                 235                 240

gcc tgc aac ggc ggt aca cta ggc ctg gat atc atc gct cat ctg cgc 973
Ala Cys Asn Gly Gly Thr Leu Gly Leu Asp Ile Ile Ala His Leu Arg
    245                 250                 255

tac ttc cag aac acc gat gcc tgc aca gcg ggc ggt atc tcg tgg aag 1021
Tyr Phe Gln Asn Thr Asp Ala Cys Thr Ala Gly Gly Ile Ser Trp Lys
260                 265                 270                 275

aga ggg gac aag gcc aag cgt gac gag atc ccc aag cgc cag gag ggc 1069
Arg Gly Asp Lys Ala Lys Arg Asp Glu Ile Pro Lys Arg Gln Glu Gly
                280                 285                 290

atg acg gat gag gag ttg gag cag aag ctc aac gac tat gtc gcc atg 1117
Met Thr Asp Glu Glu Leu Glu Gln Lys Leu Asn Asp Tyr Val Ala Met
            295                 300                 305

gac aag gag tac gtg gac agc cat aag atc tag                                    1150
Asp Lys Glu Tyr Val Asp Ser His Lys Ile
        310                 315

<210>  10

<211>  348

<212>  PRT

<213>  Fusarium solani

<400>  10

Met Arg Leu Leu Pro Leu Leu Ser Val Val Thr Leu Thr Ala Ala Ser
    -30                 -25                 -20

Pro Ile Ala Ser Val Gln Glu Tyr Thr Asp Ala Leu Glu Lys Arg Ala
-15                 -10                 -5                  -1  1

Ile Thr Ala Ser Gln Leu Asp Tyr Glu Asn Phe Lys Phe Tyr Ile Gln
            5                   10                  15

His Gly Ala Ala Ala Tyr Cys Asn Ser Glu Thr Ala Ser Gly Gln Lys
        20                  25                  30

Ile Thr Cys Asn Asp Asn Gly Cys Lys Gly Ile Glu Ala Asn Asn Ala
    35                  40                  45

Ile Ile Val Ala Ser Phe Val Gly Thr Gly Thr Gly Ile Gly Gly Tyr
50                  55                  60                  65

Val Ser Thr Asp Asn Val Arg Lys Glu Ile Val Leu Ser Ile Arg Gly
                70                  75                  80

Ser Ser Asn Ile Arg Asn Trp Leu Thr Asn Val Asp Phe Gly Gln Ser
            85                  90                  95

Ser Cys Ser Tyr Val Arg Asp Cys Gly Val His Thr Gly Phe Arg Asn
        100                 105                 110

Ala Trp Asp Glu Ile Ala Gln Arg Ala Arg Asp Ala Val Ala Lys Ala
    115                 120                 125

Arg Ala Met Asn Pro Ser Tyr Lys Val Ile Ser Thr Gly His Ser Leu
130                 135                 140                 145

Gly Gly Ala Val Ala Thr Leu Gly Ala Ala Asp Leu Arg Ser Lys Gly
                150                 155                 160

Thr Ala Val Asp Ile Phe Thr Phe Gly Ala Pro Arg Val Gly Asn Ala
            165                 170                 175

Glu Leu Ser Ala Phe Ile Thr Ala Gln Ala Gly Gly Glu Phe Arg Val
        180                 185                 190

Thr His Gly Arg Asp Pro Val Pro Arg Leu Pro Pro Ile Val Phe Gly
    195                 200                 205

Tyr Arg His Thr Ser Pro Glu Tyr Trp Leu Ala Gly Gly Ala Ser Thr
210                 215                 220                 225

Lys Ile Asp Tyr Ser Val Asn Asp Ile Lys Val Cys Glu Gly Ala Ala
                230                 235                 240

Asn Leu Ala Cys Asn Gly Gly Thr Leu Gly Leu Asp Ile Ile Ala His
            245                 250                 255

Leu Arg Tyr Phe Gln Asn Thr Asp Ala Cys Thr Ala Gly Gly Ile Ser
        260                 265                 270

Trp Lys Arg Gly Asp Lys Ala Lys Arg Asp Glu Ile Pro Lys Arg Gln
    275                 280                 285

Glu Gly Met Thr Asp Glu Glu Leu Glu Gln Lys Leu Asn Asp Tyr Val
290                 295                 300                 305

Ala Met Asp Lys Glu Tyr Val Asp Ser His Lys Ile
                310                 315

<210>  11

<211>  35

<212>  DNA

<213>  Artificial/Unknown


<220>

<221>  misc_feature

<222>  ()..()

<223>  Primer



<400>  11
cagtgaattg gcctcgatgg ccgcggccgc gaatt                                35

<210>  12

<211>  35

<212>  DNA

<213>  Artificial/Unknown

<220>

<221>  misc_feature

<222>  ()..()

<223>  Primer



<400>  12
aattcgcggc cgcggccatc gaggccaatt cactg                                35

<210>  13

<211>  34

<212>  DNA

<213>  Artificial/Unknown


<220>

<221>  misc_feature

<222>  ()..()

<223>  Primer



<400>  13
cacgaaggaa agacgatggc tttcacggtg tctg                                 34

<210>  14

<211>  34

<212>  DNA

<213>  Artificial/Unknown


<220>

<221>  misc_feature

<222>  ()..()

<223>  Primer



<400>  14
cagacaccgt gaaagccatc gtctttcctt cgtg                                 34

<210>  15

<211>  46

<212>  DNA

<213>  Artificial/Unknown

<220>

<221>  misc_feature

<222>  ()..()

<223>  Primer

<400>  15
ctatctcttc accatggtac cttaattaaa taccttgttg gaagcg                    46

<210>  16

<211>  46

<212>  DNA

<213>  Artificial/Unknown

<220>

<221>  misc_feature

<222>  ()..()

<223>  Primer

<400>  16
cgcttccaac aaggtattta attaaggtac catggtgaag agatag                    46

<210>  17

<211>  37

<212>  DNA

<213>  Artificial/Unknown

<220>

<221>  misc_feature

<222>  ()..()

<223>  Primer

<400>  17
cgttctttgt ctgtcagcat gcatctccta tcactcc                                37

<210>  18
<211>  45
<212>  DNA
<213>  Artificial/Unknown

<220>
<221>  misc_feature
<222>  ()..()
<223>  Primer

<400>  18
ccagagtttt tgttatggtt aattaatatc gttactgcgt aaatg                       45

<210>  19
<211>  20
<212>  DNA
<213>  Artificial/Unknown

<220>
<221>  misc_feature
<222>  ()..()
<223>  lip2

<400>  19
aarttytaya thcarcaygg                                                   20

<210>  20
<211>  23
<212>  DNA

<213>  Artificial/Unknown

<220>
<221>  modified_base
<222>  (1)..(23)
<223>  i

<220>
<221>  misc_feature
<222>  ()..()
<223>  Primer lip3

<400>  20
carcayggng cngcngcnta ytg                                              23

<210>  21
<211>  23
<212>  DNA
<213>  Artificial/Unknown

<220>
<221>  misc_feature
<222>  ()..()
<223>  lip10

<220>
<221>  modified_base
<222>  (1)..(23)
<223>  i

<400>  21
ggntgyggng tncaynnngg ntt                                              23

<210>  22

<211>  23

<212>  DNA

<213>  Artificial/Unknown

<220>

<221>  modified_base

<222>  (1)..(23)

<223>  i

<220>

<221>  misc_feature

<222>  ()..()

<223>  lip11

<400>  22
aanccnnnrt gnacnccrca ncc                                               23

<210>  23

<211>  20

<212>  DNA

<213>  Artificial/Unknown

<220>

<221>  misc_feature

<222>  ()..()

<223>  Primer lip15

<220>

<221>  modified_base

<222>  (1)..(20)

<223>  i

<400>  23
ccnccnarns wrtgnccngt                                                   20

<210>  24

<211>  20

<212>  DNA

<213>  Artificial/Unknown

<220>

<221>  misc_feature

<222>  ()..()

<223>  lip17

<220>

<221>  modified_base

<222>  (1)..(20)

<223>  i

<400>  24
ggrtcntyns crtkngtnac                                                   20

<210>  25

<211>  23

<212>  DNA

<213>  Artificial/Unknown

<220>

<221>  misc_feature

<222>  ()..()

<223>  lip21

<220>

<221>  modified_base
<222>  (1)..(23)
<223>  i

<400>  25
tcnswngtyt gnckrtancc raa                                              23

<210>  26
<211>  44
<212>  DNA
<213>  Artificial/Unknown

<220>
<221>  misc_feature
<222>  ()..()
<223>  Adaptor L

<400>  26
ctaatacgac tcactatagg gctcgagcgg ccgcccgggc aggt                       44

<210>  27
<211>  21
<212>  DNA
<213>  Artificial/Unknown

<220>
<221>  misc_feature
<222>  ()..()
<223>  27N1long

<400>  27
tggacaaccg ttccttgcgc a                                                21

<210>  28

<211>  25

<212>  DNA

<213>  Artificial/Unknown


<220>

<221>  misc_feature

<222>  ()..()

<223>  27C1long



<400>  28
tacacgtacg gtgctcctcg agtgg                                             25

<210>  29

<211>  20

<212>  DNA

<213>  Artificial/Unknown


<220>

<221>  misc_feature

<222>  ()..()

<223>  27C2



<400>  29
tatctggcgg cggtggcgac                                                   20

<210>  30

<211>  36

<212>  DNA

<213>  Artificial/Unknown


<220>

<221>  misc_feature

<222>  ()..()

<223>  27N(Bam)

<400>  30
cgcggatcca tgctcctcct accactcctc tcagcc                                36

<210>  31

<211>  48

<212>  DNA

<213>  Artificial/Unknown


<220>

<221>  misc_feature

<222>  ()..()

<223>  27C(Sal)



<400>  31
acgcgtcgac ttatgatgaa cgattcttat ggctatccac atactcct                   48

<210>  32

<211>  30

<212>  DNA

<213>  Artificial/Unknown


<220>

<221>  misc_feature

<222>  ()..()

<223>  AcN3



<400>  32
tggatccgcc gcacttcaca gcttgacccg                                       30

<210>  33

<211>  32

<212>  DNA

<213>  Artificial/Unknown

<220>

<221>  misc_feature

<222>  ()..()

<223>  AcC3

<400>  33
cggcaacggt gtcttctcta acttcgtcag cc                                    32

<210>  34

<211>  38

<212>  DNA

<213>  Artificial/Unknown

<220>

<221>  misc_feature

<222>  ()..()

<223>  152-N (Bcl)

<400>  34
tattatcatg atcaatgctc gccctatccc ttctttct                              38

<210>  35

<211>  36

<212>  DNA

<213>  Artificial/Unknown

<220>

<221>  misc_feature

<222>  ()..()

<223>  152-C(Xho)

<400>  35
ccgctcgagc tacaacccag ccacaaagtc cttgtc                                36

<210>  36

<211>  26

<212>  DNA

<213>  Artificial/Unknown

<220>

<221>  misc_feature

<222>  ()..()

<223>  140288

<400>  36
ttgaattcat gggtaataac tgatat                                            26

<210>  37

<211>  44

<212>  DNA

<213>  Artificial/Unknown

<220>

<221>  misc_feature

<222>  ()..()

<223>  141222

<400>  37
ggtattgtcc tgcagacggc aatttaacgg cttctgcgaa tcgc                        44

<210>  38

<211>  45

<212>  DNA

<213>  Artificial/Unknown

<220>

<221>  misc_feature

<222>  ()..()

<223>  141223

<400>  38
ggatgctgtt gactccggaa atttaacggt ttggtcttgc atccc                45

<210>  39

<211>  32

<212>  DNA

<213>  Artificial/Unknown

<220>

<221>  misc_feature

<222>  ()..()

<223>  142778

<400>  39
aaatcaatct attttcaatt caattcatca tt                              32

<210>  40

<211>  31

<212>  DNA

<213>  Artificial/Unknown

<220>

<221>  misc_feature

<222>  ()..()

<223>  142779

<400>  40
ttgaattgaa aatagattga tttaaaactt c                               31

<210>  41

<211>  25

<212>  DNA

<213>  Artificial/Unknown

<220>

<221>  misc_feature

<222>  ()..()

<223>  142780

<400>  41
ttgcatgcgt aatcatggtc atagc                                          25

<210>  42

<211>  30

<212>  DNA

<213>  Artificial/Unknown

<220>

<221>  misc_feature

<222>  ()..()

<223>  12N1

<400>  42
actgaacagt tggacagccg tttccgctgc                                     30

<210>  43

<211>  32

<212>  DNA

<213>  Artificial/Unknown

<220>

<221>  misc_feature

<222>  ()..()

<223>  12C2

<400>  43
ccaggctggt ggagagttcc gcgttacgaa cg                                  32

<210>  44

<211>  33

<212>  DNA

<213>  Artificial/Unknown

<220>

<221>  misc_feature

<222>  ()..()

<223>  12-N (Bcl)

<400>  44
ttgtctgtga tcatgcgtct cctgtcactc ctc                                    33

<210>  45

<211>  44

<212>  DNA

<213>  Artificial/Unknown

<220>

<221>  misc_feature

<222>  ()..()

<223>  12-C (Sal)

<400>  45
ttagtgcgta aacagctgac tatgatgagc ggctggcgtg agtc                        44

<210>  46

<211>  23

<212>  DNA

<213>  Artificial/Unknown

<220>

<221>  misc_feature

<222>  ()..()

<223>  161000j1

<400>  46
acaggccact cccttggagg ngc                                              23

<210>  47

<211>  24

<212>  DNA

<213>  Artificial/Unknown

<220>

<221>  misc_feature

<222>  ()..()

<223>  161000j2

<400>  47
aggagggaga cgggggacng grtc                                             24

<210>  48

<211>  28

<212>  DNA

<213>  Artificial/Unknown

<220>

<221>  misc_feature

<222>  ()..()

<223>  071200j1

<400>  48
gctgaactct cagcattcat cacggccc                                         28

<210>  49

<211>  28

<212>  DNA

<213>  Artificial/Unknown

<220>

<221>  misc_feature

<222>  ()..()

<223>  071200j2

<400>  49
ccaaaggtga agatgtcaac tgcggttc                                          28

<210>  50

<211>  26

<212>  DNA

<213>  Artificial/Unknown

<220>

<221>  misc_feature

<222>  ()..()

<223>  221200J1

<400>  50
cggcggcgag ttccgtgtca ctcatg                                            26

<210>  51

<211>  25

<212>  DNA

<213>  Artificial/Unknown

<220>

<221>  misc_feature

<222>  ()..()

<223>  221200J2

<400>  51
gacctcaggt cagcggcacc cagag                                             25

<210>  52

<211>  28

<212>  DNA

<213>  Artificial/Unknown

<220>

<221>  misc_feature

<222>  ()..()

<223>  170101J11

<400>  52
cttgaagttt tcatagtcaa gttgagag                                          28

<210>  53

<211>  39

<212>  DNA

<213>  Artificial/Unknown

<220>

<221>  misc_feature

<222>  ()..()

<223>  290101j2

<400>  53
actagcctcg agctagatct tatggctgtc cacgtactc                              39

<210>  54

<211>  45

<212>  DNA

<213>  Artificial/Unknown

<220>

<221>  misc_feature

<222>  ()..()

<223>  020301j1

<400>  54
gcgcgcggat ccaccatgcg cctgcttcct ctcctctcgg tcgta                        45

<210>  55

<211>  21

<212>  DNA

<213>  Artificial/Unknown

<220>

<221>  misc_feature

<222>  ()..()

<223>  19670

<400>  55
ccccatcctt taactatagc g                                                  21

<210>  56

<211>  24

<212>  DNA

<213>  Artificial/Unknown

<220>

<221>  misc_feature

<222>  ()..()

<223>  19671

<400>  56
ctcccttctc tgaacaataa accc                                               24

<210>  57

<211>  11

<212>  DNA

<213>  Artificial/Unknown

<220>

<221>  misc_feature

<222>  ()..()

<223>  nucleotides 134-144



<400>  57
gtactaaaac c                                                            11

<210>  58

<211>  11

<212>  DNA

<213>  Artificial/Unknown


<220>

<221>  misc_feature

<222>  ()..()

<223>  134-144 altered



<400>  58
ccgttaaatt t                                                            11

<210>  59

<211>  0

<212>  DNA

<213>  Artificial/Unknown


<220>

<221>  misc_feature

<222>  ()..()

<223>  nucleotides 423-436



<400>  59
000

<210>  60

<211>  14

<212>  DNA

<213>  Artificial/Unknown

<220>

<221>  misc_feature

<222>  ()..()

<223>  423-436 altered

<400>  60
cggcaattta acgg                                                          14

## Description

### FIELD OF THE INVENTION

The present invention relates to a polynucleotide encoding a lipolytic enzyme and to a lipolytic enzyme. The invention also relates to methods of producing and using the lipolytic enzyme.

### BACKGROUND OF THE INVENTION

Lipolytic enzymes (such as lipases and phospholipases) are capable of hydrolyzing carboxylic ester bonds in a substrate to release carboxylic acids. They are known to be useful, e.g., in baking and detergents.

Various species of the related genera *Acremonium* and *Fusarium* are known to produce lipolytic enzymes. Thus, Roberts et al., Mycologia, 79, 265-273, 1987. Satyanarayana & Johri, Current Science, 50, 680-682, 1981 state that some isolates of *Acremonium* produce lipase.

WO 98/26057 discloses a polypeptide having lipase and phospholipase activity (GenBank Acc. No. A85215) obtained from *Fusarium oxysporum.* A lipase from *Fusarium heterosporum* and its sequence are known. Journal of Fermentation and Bioengineering, 75 (5), p 349-352, 1993. Journal of Biochemistry (Tokyo), 116 (3), p 536-540, 1994. A lipolytic enzyme from *Fusarium culmorum* CBS 513.94 and its N-terminal sequence are disclosed in US 5830736. A lipase/phospholipase from *Fusarium oxysporum* and its sequence are disclosed in WO 98/26057. US 5990069 discloses a lipase from a strain of *Fusarium solani var. minus.*

### SUMMARY OF THE INVENTION

The inventors have isolated a group of genes encoding lipolytic enzymes with high homology from *Fusarium* and *Acremonium.*

Accordingly, one aspect of the invention provides a polynucleotide encoding a lipolytic enzyme which comprises:
a) the DNA sequence encoding a mature lipolytic enzyme cloned into a plasmid present in *Escherichia coli* DSM 13539,
b) the mature polypeptide-encoding DNA sequence shown in SEQ ID NO: 3;
c) an analogue of the DNA sequence defined in a) or b) which
   i) has at least 80% identity with said DNA sequence, or
   ii) hybridizes at high stringency with said DNA sequence, its complementary strand or a subsequence thereof.

Another aspect of the invention also provides a lipolytic enzyme which may be a polypeptide having an amino acid sequence as the mature peptide shown in SEQ ID NO: 2, 4, 6, 8 or 10. It may be C-terminally processed to consist of amino acids 1-282 of SEQ ID NO: 2, 1-282 of SEQ ID NO: 4, 1-275 of SEQ ID NO: 6, 1-282 of SEQ ID NO: 8 or 1-276 of SEQ ID NO: 10.

Further, the lipolytic enzyme of the invention may be a polypeptide encoded by the lipolytic enzyme encoding part of the DNA sequence cloned into a plasmid present in *Escherichia coli* deposit number DSM 13539.

The lipolytic enzyme may also be an analogue of the polypeptide defined above which:
i) has at least 85% identity with said polypeptide,
ii) is immunologically reactive with an antibody raised against said polypeptide in purified form,
iii) is an allelic variant of said polypeptide,

Finally, the lipolytic enzyme of the invention may be a polypeptide which is encoded by a nucleic acid sequence which hybridizes under high stringency conditions with a complementary strand of the mature polypeptide-encoding nucleic acid sequence of SEQ ID NO: 1, 3, 5, 7 or 9 or a subsequence thereof having at least 100 nucleotides.

A further aspect of the invention provides a nucleic acid sequence comprising a nucleic acid sequence which encodes the lipolytic enzyme described above.

Other aspects of the invention provide a recombinant expression vector comprising the DNA sequence, and a cell transformed with the DNA sequence or the recombinant expression vector.

The DNA sequences of the invention have the following identities to known DNA sequences:

| **SEQ ID NO:** | **Source organism** | **Closest known sequence** | **DNA identity** |
|---|---|---|---|
| 1 | *F. venenatum* | *F. oxysporum* | 79 % |
| *3* | *F. sulphureum* | *F. oxysporum* | 77 % |
| *5* | *A. berkeleyanum* | *F. heterosporum* | 68 % |
| *7* | *F. culmorum* | *F. oxysporum* | 79 % |
| *9* | *F. solani* | *F. oxysporum* | 68 % |

### DETAILED DESCRIPTION OF THE INVENTION

### Genomic DNA source

A lipolytic enzyme of the invention may be derived from a strain of *Fusarium* or *Acremonium,* particularly *F. venenatum, F. sulphureum, A. berkeleyanum, F. culmorum* or *F. solani,* using probes designed on the basis of the DNA sequences in this specification, or from a Plasmid contained in an *E. coli* strain identified below.

More particularly, the *Fusarium venenatum* cell may be *Fusarium venenatum* A3/5, which was originally deposited as *Fusarium graminearum* ATCC 20334 and recently reclassified as *Fusarium venenatum* by Yoder and Christianson, 1998, Fungal Genetics and Biology 23: 62-80 and O'Donnell et al., 1998, Fungal Genetics and Biology 23: 57-67. Alternatively, the *Fusarium venenatum* cell may be a morphological mutant of *Fusarium venenatum* A3/5 or *Fusarium venenatum* ATCC 20334, as disclosed in WO 97/26330. ATCC 20334 is available on commercial terms from American Type Culture Collection, 10801 University Boulevard, Manassas, Virginia 20110-2209 ("ATCC").

Other strains are *A. berkeleyanum* CBS 301.38, *F. culmorum* CBS 513.94 and *F. solani* MUCL 38667. The two CBS strains are available on commercial terms from Centraalbureau voor Schimmelcultures, Uppsalalaan 8, 3584 CT Utrecht, the Netherlands (P.O.Box 85167, 3508 AD Utrecht, the Netherlands). MUCL 38667 is available on commercial terms from Mycothèque de l'Université Catholique de Louvain, Place Croix du Sud 3, B-1348 Louvain-la-Neuve, Belgium by referring to US 5990069.

Strains of *Escherichia coli* each containing a gene encoding a lipolytic enzyme from a source organism were deposited by the inventors under the terms of the Budapest Treaty as follows:

| **Source organism** | ***E. coli* deposit date** | ***E. coli* deposit number** |
|---|---|---|
| *F. venenatum* WTY700 3.8d | 22 August 2000 | NRRL B-30333 |
| | (Resubmitted on 30 | |
| | March 2001) | |
| *F.sulphureum* | 15 June 2000 | DSM 13539 |
| *A. berkeleyanum* CBS 301.38 | 15 June 2000 | DSM 13538 |
| *F. culmorum* CBS 513.94 | 15 June 2000 | DSM 13537 |
| *Fusarium solani* MUCL 38667 | 21 June 2001 | DSM 14361 |

NRRL indicates a deposit made at Agricultural Research Service Patent Culture Collection, Northern Regional Research Center, 1815 University Street, Peoria, Illinois, 61604. NRRL B-30333 was resubmitted on 30 March 2001. DSM indicates a deposit made at DSMZ - Deutshe Sammmlung von Microorganismen und Zellkulturen GmbH, Mascheroder Weg 1 b, D-38124 Braunschweig DE, Germany.

The deposits DSM 13537, DSM 13538 and DSM 13539 were made by Novo Nordisk A/S and were later assigned to Novozymes A/S.

### Polypeptide and polynucleotide sequences

The lipolytic enzyme of the invention may be a polypeptide having the amino acid sequence shown in SEQ ID NO: 2, 4, 6, 8 or 10 or it may be an amino acid variant derived from any of these sequences by substitution, deletion and/or insertion of one or more amino acid residues. The variant may be constructed in analogy with known variants of other lipases from *Fusarium,* e.g. from *F. oxysporum* and *F*. *heterosporum* as disclosed in WO 200032758 and T. Nagao, Journal of Bioscience and Bioengineering, 89 (5), 446-450 (2000).

The lipolytic enzyme may be C-terminally processed, e.g. by KEX-2 hydrolysis of the bond after Arg and Lys corresponding to K274 and R275 of SEQ ID NO: 6 to remove a peptide at the C-terminal. The C-terminally processed polypeptide tends to have a lower thermostability than the full-length polypeptide.

The variant may have substitutions of amino acids Arg and/or Lys corresponding to K274 and/or R275 of SEQ ID NO: 6, e.g. R275A and/or K274A. Such variants may be protected against C-terminal processing by KEX-2 hydrolysis to preserve the full-length polypeptide with higher thermostability.

A polynucleotide sequence encoding any of the polypeptides described above may be constructed by principles known in the art.

### Properties of lipolytic enzyme

The lipolytic enzyme is able to hydrolyze carboxylic ester bonds and is classified as EC 3.1.1 according to Enzyme Nomenclature 1992, Academic Press, Inc. The enzyme has lipase (triacylglycerol lipase) activity (EC 3.1.1.3) and may also have other activities such as phospholipase activity, particularly phospholipase A1 (EC 3.1.1.32), and/or galactolipase (EC 3.1.1.26).

### Recombinant expression vector

The expression vector of the invention typically includes control sequences encoding a promoter, operator, ribosome binding site, translation initiation signal, and, optionally, a selectable marker, a transcription terminator, a repressor gene or various activator genes. The vector may be an autonomously replicating vector, or it may be integrated into the host cell genome.

### Production by cultivation of transformant

The lipolytic enzyme of the invention may be produced by transforming a suitable host cell with a DNA sequence encoding the lipolytic enzyme, cultivating the transformed organism under conditions permitting the production of the enzyme, and recovering the enzyme from the culture.

The host organism may particularly be a eukaryotic cell, in particular a fungal cell, such as a yeast cell or a filamentous fungal cell, e.g. a strain of *Aspergillus, Fusarium, Trichoderma* or *Saccharomyces,* particularly *A. niger, A. oryzae, F. graminearum, F. sambucinum, F. cerealis* or *S*. *cerevisiae.* The production of the lipolytic enzyme in such host organisms may be done by the general methods described in EP 238,023 (Novo Nordisk), WO 96/00787 (Novo Nordisk) or EP 244,234 (Alko).

### DNA recombination (shuffling)

The invention provides production of a lipolytic enzyme by shuffling of at least two polynucleotides, expressing the shuffled polynucleotides to form recombinant polypeptides, screening the polypeptides to select a polypeptide having lipolytic enzyme activity, and producing the selected polypeptide.

The polynucleotides to be shuffled include a first polynucleotide comprising the and a second different polynucleotide encoding a lipolytic enzyme and having at least 50% identity to the first polynucleotide. The second polynucleotide may comprise a second mature polypeptide-coding sequence of SEQ ID NO: 1, 3, 5, 7 or 9 or may comprise a polynucleotide encoding a lipolytic enzyme from *F. oxysporum* (WO 98/26057) or from *F. heterosporum.*

Shuffling (or recombination) of the homologous input polynucleotides (starting-point polynucleotides) may involve fragmenting the polynucleotides and recombining the fragments, to obtain output polynucleotides (i.e. polynucleotides that have been subjected to a shuffling cycle) wherein a number of nucleotide fragments are exchanged in comparison to the input polynucleotides.

DNA recombination or shuffling may be a (partially) random process in which a library of chimeric genes is generated from two or more starting genes. A number of known formats can be used to carry out this shuffling or recombination process.

The process may involve random fragmentation of parental DNA followed by reassembly by PCR to new full length genes, e.g. as presented in US5605793, US5811238, US5830721, US6117679. In-vitro recombination of genes may be carried out, e.g. as described in US6159687, WO98/41623, US6159688, US5965408, US6153510. The recombination process may take place *in vivo* in a living cell, e.g. as described in WO 97/07205 and WO 98/28416.

The parental DNA may be fragmented by DNA'se I treatment or by restriction endonuclease digests as described by Kikuchi et al (Gene 236:159-167, 2000). Shuffling of two parents may be done by shuffling single stranded parental DNA of the two parents as described in Kikuchi et al (Gene 243:133-137, 2000).

A particular method of shuffling is to follow the methods described in Crameri et al, 1998, Nature, 391: 288-291 and Ness et al. Nature Biotechnology 17: 893-896. Another format would be the methods described in US 6159687: example 1 and 2.

### Hybridization

The hybridization is used to indicate that a given DNA sequence is analogous to a nucleotide probe corresponding to a DNA sequence of the invention. The hybridization conditions are described in detail below.

Suitable conditions for determining hybridization between a nucleotide probe and a homologous DNA or RNA sequence involves presoaking of the filter containing the DNA fragments or RNA to hybridize in 5x SSC (standard saline citrate) for 10 min, and prehybridization of the filter in a solution of 5x SSC (Sambrook et al. 1989), 5x Denhardt's solution (Sambrook et al. 1989), 0.5% SDS and 100 µg/ml of denatured sonicated salmon sperm DNA (Sambrook et al. 1989), followed by hybridization in the same solution containing a random-primed (Feinberg, A. P. and Vogelstein, B. (1983) Anal. Biochem. 132:6-13), ³²P-dCTP-labeled (specific activity > 1 x 10⁹ cpm/µg) probe for 12 hours at approx. 45°C. The filter is then washed two times for 30 minutes in 2x SSC, 0.5% SDS at a temperature of at least 55°C, more particularly at least 60°C, more particularly at least 65°C, even more particularly at least 70°C, especially at least 75°C.

Molecules to which the oligonucleotide probe hybridizes under these conditions are detected using a x-ray film.

### Alignment and identity

The lipolytic enzyme and the nucleotide sequence of the invention may have homologies to the disclosed sequences of at least 85%, particularly at least 90% or at least 95%, e.g. at least 98%.

For purposes of the present invention, alignments of sequences and calculation of identity scores were done using a Needleman-Wunsch alignment (i.e. global alignment), useful for both protein and DNA alignments. The default scoring matrices BLOSUM50 and the identity matrix are used for protein and DNA alignments respectively. The penalty for the first residue in a gap is -12 for proteins and -16 for DNA, while the penalty for additional residues in a gap is -2 for proteins and -4 for DNA. Alignment is from the FASTA package version v20u6 (W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA", Methods in Enzymology, 183:63-98).

### Lipase activity (LU)

A substrate for lipase is prepared an emulsion of 5% by volume of tributyrin (glycerin tributyrate) using 0.1% gum Arabic as emulsifier. The hydrolysis of tributyrin at 30°C at pH 7 is followed in a pH-stat titration experiment. One unit of lipase activity (1 LU) equals the amount of enzyme capable of releasing 1 µmol butyric acid/min at the standard conditions. 1 KLU = 1000 LU.

### Use of lipolytic enzyme

The lipolytic enzyme of the invention can be used in various industrial applications of lipolytic enzymes, e.g. in baking or detergents as described below. It can also be used for diglyceride synthesis (EP 307154), acidolysis, interesterification (WO 8802775), ester hydrolysis, oil degumming (JP-A 2-153997, US 5264367), production of lysolecithin (JP patent 2794574, JP-B 6-087751) and in a cheese-making process (WO 0054601).

### Use in baking

The lipolytic enzyme of the invention can be used in the preparation of dough, bread and cakes, e.g. to improve the elasticity of the bread or cake. Thus, the lipolytic enzyme can be used in a process for making bread, comprising adding the lipolytic enzyme to the ingredients of a dough, kneading the dough and baking the dough to make the bread. This can be done in analogy with WO 9404035 and EP 585988. The lipolytic enzyme is typically added in an amount corresponding to 0.01-100 mg enzyme protein per kg of flour, particularly 0.1-25 mg enzyme protein per kg of flour, more particularly 0.1-10 mg enzyme protein per kg of flour, and most particularly 0.5-5 mg enzyme protein per kg of flour.

### Use in detergent

The lipolytic enzyme may be used as a detergent additive, e.g. at a concentration (expressed as pure enzyme protein) of 0.001-10 (e.g. 0.01-1) mg per gram of detergent or 0.001-100 (e.g. 0.01-10) mg per liter of wash liquor.

The detergent composition of the invention may for example be formulated as a hand or machine laundry detergent composition including a laundry additive composition suitable for pre-treatment of stained fabrics and a rinse added fabric softener composition, or be formulated as a detergent composition for use in general household hard surface cleaning operations. In a laundry detergent, the lipolytic enzyme may be effective for the removal of fatty stains, for whiteness maintenance and for dingy cleanup. A laundry detergent composition may be formulated as described in WO 97/04079, WO 97/07202, WO 97/41212, PCT/DK WO 98/08939 and WO 97/43375.

The detergent composition of the invention may particularly be formulated for hand or machine dishwashing operations. e.g. as described in GB 2,247,025 (Unilever) or WO 99/01531 (Procter & Gamble). In a dishwashing composition, the lipolytic enzyme may be effective for removal of greasy/oily stains, for prevention of the staining /discoloration of the dishware and plastic components of the dishwasher by highly colored components and the avoidance of lime soap deposits on the dishware.

Some embodiments of the invention are described in the following paragraphs:
Paragraph 1: A polynucleotide which comprises a nucleotide sequence encoding a lipolytic enzyme, which sequence:
   a) is a DNA sequence cloned into a plasmid present in *Escherichia coli* deposit number *Escherichia coli* NRRL B-30333, DSM 13537, DSM 13538, DSM 13539 or DSM 14361;
   b) is the mature polypeptide-encoding DNA sequence shown in SEQ ID NO: 1, 3, 5, 7 or 9 or is a DNA sequence that can be derived there from by substitution, deletion, and/or insertion of one or more nucleotides; or
   c) has at least 80% identity with the mature polypeptide-encoding sequence of SEQ ID NO: 1, 3 or 7, or at least 70% identity with the mature polypeptide-encoding sequence of SEQ ID NO: 5 or 9; or
   d) a complementary strand of the sequence defined in a), b) or c).
Paragraph 2: The polynucleotide of paragraph 1 which is native to a strain of *Fusarium* or *Acremonium,* particularly *F. venenatum, F. sulphureum, A. berkeleyanum, F. culmorum* or *F. solani.*
Paragraph 3: A polypeptide having lipolytic enzyme activity which is:
   a) a polypeptide having an amino acid sequence which has at least 92% identity with the mature polypeptide of SEQ ID NO: 2, at least 85% identity with the mature polypeptide of SEQ ID NO: 4 or at least 70% identity with the mature polypeptide of SEQ ID NO: 6;
   b) a polypeptide which is encoded by a nucleic acid sequence which hybridizes under high stringency conditions with a complementary strand of the nucleic acid sequence encoding the mature polypeptide of SEQ ID NO: 1, 3 or 5 or a subsequence thereof having at least 100 nucleotides;
   c) a polypeptide having an amino acid sequence which can be obtained from the mature polypeptide of SEQ ID NO: 2, 4 or 6 by substitution, deletion, and/or insertion of one or more amino acids; or
   d) a polypeptide encoded by the lipolytic enzyme encoding part of the DNA sequence cloned into a plasmid present in *Escherichia coli* deposit number NRRL B-30333, DSM 13538 or DSM 13539, or DSM 14361.
Paragraph 4: The lipolytic enzyme of paragraph 3 which is native to a strain of *Fusarium* or *Acremonium,* particularly *F. venenatum, F. sulphureum* or *A. berkeleyanum.*
Paragraph 5: The lipolytic enzyme of paragraph 3 or 4 which has an amino acid sequence comprising the mature polypeptide of SEQ ID NO: 2, 4 or 6.
Paragraph 6: A polynucleotide comprising a nucleic acid sequence which encodes the lipolytic enzyme of any of paragraphs 3-5.
Paragraph 7: A nucleic acid construct comprising the polynucleotide of paragraph 1, 2 or 6 operably linked to one or more control sequences capable of directing the expression of the lipolytic enzyme in a suitable expression host.
Paragraph 8: A recombinant expression vector comprising the nucleic acid construct of paragraph 7, a promoter, and transcriptional and translational stop signals.
Paragraph 9: A recombinant host cell comprising the nucleic acid construct of paragraph 7.
Paragraph 10: A method for producing a lipolytic enzyme comprising cultivating the host cell of paragraph 10 under conditions conducive to production of the lipolytic enzyme, and recovering the lipolytic enzyme.
Paragraph 11: A method for producing a mutant nucleic acid sequence, comprising
   a) introducing at least one mutation into the mature polypeptide coding sequence of SEQ ID NO: 1, 3, 5, 7 or 9; and
   b) recovering the mutant nucleic acid sequence.
Paragraph 12: A method for producing a polypeptide comprising
   a) introducing at least one mutation into a polynucleotide having the mature polypeptide coding sequence of SEQ ID NO: 1, 3, 5, 7 or 9;
   b) transforming a host cell with the mutated polynucleotide;
   c) cultivating the transformed host cell under conditions conducive for production of polypeptides;
   d) screening the polypeptides to select a polypeptide having lipolytic enzyme activity; and
   e) producing the selected polypeptide.
Paragraph 13: A method of producing a lipolytic enzyme which comprises:
   a) shuffling a first polynucleotide comprising the mature polypeptide coding sequence of SEQ ID NO: 1, 3, 5, 7 or 9 and a second polynucleotide encoding a lipolytic enzyme and having at least 50% identity to the first polynucleotide;
   b) expressing the shuffled polynucleotides to form recombinant polypeptides;
   c) screening the polypeptides to select a polypeptide having lipolytic enzyme activity; and
   d) producing the selected polypeptide.
Paragraph 14: A method for preparing a dough or a baked product made from the dough, comprising adding the lipolytic enzyme of any of paragraphs 3-5 to the dough.
Paragraph 15: A dough composition comprising the lipolytic enzyme of any of paragraphs 3-5.
Paragraph 16: A detergent composition comprising a surfactant and the lipolytic enzyme of any of paragraphs 3-5.

### MATERIALS AND METHODS

### Methods

Unless otherwise stated, DNA manipulations and transformations were performed using standard methods of molecular biology as described in Sambrook et al. (1989) Molecular cloning: A laboratory manual, Cold Spring Harbor lab., Cold Spring Harbor, NY; Ausubel, F. M. et al. (eds.) "Current protocols in Molecular Biology", John Wiley and Sons, 1995; Harwood, C. R., and Cutting, S. M. (eds.) "Molecular Biological Methods for Bacillus". John Wiley and Sons, 1990.

### Enzymes

Enzymes for DNA manipulations (e.g. restriction endonucleases, ligases etc.) are obtainable from New England Biolabs, Inc. and were used according to the manufacturer's instructions.

### Plasmids/vectors

pT7Blue (Invitrogen, Netherlands)

pCaHj483 is described in WO 9704079 and WO 9942566.

### Cloning

LA PCR^{™} in vitro Cloning Kit (TaKaRa) was used for cloning and was used according to the manufacturer's instructions.

### Microbial strains

*Fusarium venenatum* WTY700 3.8d, a spore-purified *tri*5-minus, *dps*1-minus strain, was used as the recipient strain for transformation experiments. *Fusarium venenatum* WTY700 3.8d is a morphological mutant of *Fusarium venenatum* strain ATCC 20334 (Wiebe et al., 1991, Mycol. Research 95: 1284-1288),
*E. coli* JM109 (TOYOBO, Japan)
*E. coli* JM110 (Invitrogen)

*A. oryzae* BECh-2 is described in Danish patent application PA 1999 01726. It is a mutant of JaL 228 (described in WO 98/12300) which is a mutant of IFO 4177.

### Media and reagents

Cove: 342.3 g/L Sucrose, 20 ml/L COVE salt solution, 10mM Acetamide, 30 g/L noble agar.

Cove-2: 30 g/L Sucrose, 20 ml/L COVE salt solution, 10mM, Acetamide, 30 g/L noble agar.

Cove salt solution: per liter 26 g KCI, 26 g MgSO4-7aq, 76 g KH2PO4, 50ml Cove trace metals.

Cove trace metals: per liter 0.04 g NaB4O7-10aq, 0.4 g CuSO4-5aq, 1.2 g FeS04-7aq, 0.7 g MnSO4-aq, 0.7 g Na2MoO2-2aq, 0.7 g ZnSO4-7aq.

AMG trace metals: per liter 14.3 g ZnSO4-7aq, 2.5 g CuSO4-5aq, 0.5 g NiCI2, 13.8 g FeS04, 8.5 g MnSO4, 3.0 g citric acid.

YPG: 4 g/L Yeast extract, 1 g/L KH2PO4, 0.5 g/L MgSO4-7aq, 5 g/L Glucose, pH 6.0.

STC: 0.8 M Sorbitol, 25 mM Tris pH 8, 25 mM CaCl2.

STPC: 40 % PEG4000 in STC buffer.

Cove top agarose: 342.3 g/L Sucrose, 20 ml/L COVE salt solution, 10mM Acetamide, 10 g/L low melt agarose.

MS-9: per liter 30 g soybean powder, 20 g glycerol, pH 6.0.

MDU-pH5: per liter 45 g maltose-1aq, 7 g yeast extract, 12 g KH2PO4, 1 g MgSO4-7aq, 2 g K2SO4, 0.5 ml AMG trace metal solution and 25 g 2-morpholinoethanesulfonic acid, pH 5.0.

### Measurement of dough and breads properties

The crumb firmness was measured using a texture analyzer TA-XT2 from Stable Micro Systems.

Texture was measured according to a modified ACCA method (American Cereal Chemists' Association).

Elasticity is a measure of the degree to which a dough can be stretched without tearing. It is evaluated by rolling a piece of dough (about 30 g) to a size of about 10 cm, and having a test panel carefully pulling at opposite ends to judge the resistance and elasticity. The results are expressed on a scale from 0 (low/weak elasticity) to 10 (high/strong elasticity) with the control (dough without enzyme addition) taken as 5.

Crumb structure is determined as the property of a baked product with finer and/or thinner cell walls in the crumb and/or more uniform/homogenous distribution of cells in the crumb and is usually evaluated empirically by the skilled test baker.

The shape factor is taken as the ratio between the height and diameter of rolls after baking (average of 10 rolls).

The specific volume is calculated as volume ml per g bread, taking the mean value of the volumes of 4 loaves measured using the traditional rape seed method. The specific volume of the control (without enzyme) is defined as 100. The relative specific volume index is the percentage of the specific volume of 4 loaves per the specific volume of 4 control loaves.

### EXAMPLES

### Example 1: Fermentation and mycelial tissue from F. venenatum

*Fusarium venenatum* WTY700 3.8d was grown in a two-liter lab-scale fermentor using a fed-batch fermentation scheme with NUTRIOSE™ (Roquette Freres, S.A., Beinheim, France) as the carbon source and yeast extract. Ammonium phosphate was provided in the feed. The fermentation was maintained at pH 6-6.5 and 30°C with positive dissolved oxygen.

Mycelial samples were harvested at 2, 4, 6, and 8 days post-inoculum and quick-frozen in liquid nitrogen. The samples were stored at -80°C until they were disrupted for RNA extraction.

### Example 2: cDNA library construction

Total cellular RNA was extracted from the mycelial samples described in Example 1 according to the method of Timberlake and Barnard (1981, Cell 26: 29-37), and the RNA samples were analyzed by Northern hybridization after blotting from 1% formaldehyde-agarose gels (Davis et al., 1986, Basic Methods in Molecular Biology, Elsevier Science Publishing Co., Inc., New York). Polyadenylated mRNA fractions were isolated from total RNA with an mRNA Separator Kit™ (Clontech Laboratories, Inc., Palo Alto, CA) according to the manufacturer's instructions. Double-stranded cDNA was synthesized using approximately 5 µg of poly(A)+ mRNA according to the method of Gubler and Hoffman (1983, Gene 25: 263-269) except a *Notl*-(dT)18 primer (Pharmacia Biotech, Inc., Piscataway, NJ) was used to initiate first strand synthesis. The cDNA was treated with mung bean nuclease (Boehringer Mannheim Corporation, Indianapolis, IN) and the ends were made blunt with T4 DNA polymerase (New England Biolabs, Beverly, MA).

The cDNA was digested with *Not*l, size selected by agarose gel electrophoresis (ca. 0.7-4.5 kb), and ligated with pZErO-2.1 (Invitrogen Corporation, Carlsbad, CA) which had been cleaved with *Not*l plus *Eco*RV and dephosphorylated with calf-intestine alkaline phosphatase (Boehringer Mannheim Corporation, Indianapolis, IN). The ligation mixture was used to transform competent *E. coli* TOP10 cells (Invitrogen Corporation, Carlsbad, CA). Transformants were selected on 2YT agar plates (Miller, 1992, A Short Course in Bacterial Genetics. A Laboratory Manual and Handbook for Escherichia coli and Related Bacteria, Cold Spring Harbor Press, Cold Spring Harbor, New York) which contained kanamycin at a final concentration of 50 µg/ml.

### Example 3: Template preparation and nucleotide sequencing

From the cDNA library described in Example 2, 1192 transformant colonies were picked directly from the transformation plates into 96-well microtiter dishes which contained 200 µl of 2YT broth (Miller, 1992, *supra)* with 50 µg/ml kanamycin. The plates were incubated overnight at 37°C without shaking. After incubation 100 µl of sterile 50% glycerol was added to each well. The transformants were replicated into secondary, deep-dish 96-well microculture plates (Advanced Genetic Technologies Corporation, Gaithersburg, MD) containing 1 ml of Magnificent Broth™ (MacConnell Research, San Diego, CA) supplemented with 50 µg of kanamycin per ml in each well. The primary microtiter plates were stored frozen at -80°C. The secondary deep-dish plates were incubated at 37°C overnight with vigorous agitation (300 rpm) on rotary shaker. To prevent spilling and cross-contamination, and to allow sufficient aeration, each secondary culture plate was covered with a polypropylene pad (Advanced Genetic Technologies Corporation, Gaithersburg, MD) and a plastic microtiter dish cover.

DNA was isolated from each well using the 96-well Miniprep Kit protocol of Advanced Genetic Technologies Corporation (Gaithersburg, MD) as modified by Utterback et al. (1995, Genome Sci. Technol. 1: 1-8). Single-pass DNA sequencing (EST) was done with a Perkin-Elmer Applied Biosystems Model 377 XL Automated DNA Sequencer (Perkin-Elmer/Applied Biosystems, Inc., Foster City, CA) using dye-terminator chemistry (Giesecke et al., 1992, Journal of Virology Methods 38: 47-60) and the reverse lac sequencing primer.

### Example 4: Analysis of DNA sequence data

Nucleotide sequence data were scrutinized for quality, and samples giving improper spacing or ambiguity levels exceeding 3% were discarded or re-run. Vector sequences and ambiguous base calls at the ends of the DNA sequences were trimmed with assistance of FACTURA™ software (Perkin-Elmer Applied Biosystems, Inc., Foster City, CA. All sequences were compared to each other to determine multiplicity using AutoAssembler™ software (Perkin-Elmer Applied Biosystems, Inc., Foster City, CA). Lastly, all sequences were translated in three frames and searched against a non-redundant database (NRDB) using GeneAssist™ software (Perkin-Elmer Applied Biosystems, Inc., Foster City, CA) with a modified Smith-Waterman algorithm using the BLOSUM 62 matrix with a threshold score of 70. The NRDB was assembled from Genpept, Swiss-Prot, and PIR databases.

### Example 5: Identification of lipase cDNA clones

Putative lipase clones were identified by comparing the deduced amino acid sequence of the ESTs to protein sequences deposited in publicly available databases such as Swissprot, Genpept, and PIR using a modified Smith-Waterman search algorithm (Perkin-Elmer Applied Biosystems, Foster City, CA). Tentative identification was based on amino acid sequence similarity to numerous fungal lipases. One clone, *Fusarium venenatum* EST FA0726, was selected for nucleotide sequence analysis which revealed that the cDNA clone was truncated at its 5 prime end.

### Example 6: Fusarium venenatum genomic DNA extraction

*Fusarium venenatum* WTY700 was grown for 24 hours at 28°C and 150 rpm in 25 ml of YEG medium composed per liter of 5 g of yeast extract and 20 g of glucose. Mycelia were then collected by filtration through Miracloth (Calbiochem, La Jolla, CA) and washed once with 25 ml of 10 mM Tris-1 mM EDTA (TE) buffer. Excess buffer was drained from the mycelia which were subsequently frozen in liquid nitrogen. The frozen mycelia were ground to a fine powder in an electric coffee grinder, and the powder was added to 20 ml of TE buffer and 5 ml of 20% w/v sodium dodecylsulfate (SDS) in a disposable plastic centrifuge tube. The mixture was gently inverted several times to ensure mixing, and extracted twice with an equal volume of phenol:chloroform:isoamyl alcohol (25:24:1 v/v/v). Sodium acetate (3 M solution) was added to give a final concentration of 0.3 M and the nucleic acids were precipitated with 2.5 volumes of ice cold ethanol. The tube was centrifuged at 15,000 x g for 30 minutes and the pellet was allowed to air dry for 30 minutes before resuspension in 0.5 ml of TE buffer. DNase-free ribonuclease A was added to a concentration of 100 µg/ml and the mixture was incubated at 37°C for 30 minutes. Proteinase K (200 µg/ml) was then added and the mixture was incubated an additional hour at 37°C. Finally, the mixture was extracted twice with phenol:chloroform:isoamyl alcohol (25:24:1 v/v/v) before precipitating the DNA with sodium acetate and ethanol according to standard procedures. The DNA pellet was dried under vacuum, resuspended in TE buffer, and stored at 4°C.

### Example 7: Genomic DNA library construction, screening, and isolation of genomic Lipase clone

Genomic libraries of *Fusarium venenatum* WTY700 were constructed in λZipLox according to the manufacturer's instructions (Life Technologies, Gaithersburg, MD). *Fusarium venenatum* genomic DNA was partially digested with *Tsp*509I and size-fractionated on 1% agarose gels. DNA fragments migrating in the size range 3-7 kb were excised and eluted from the agarose gel slices using Prep-a-Gene reagents (BioRad, Hercules, CA). The eluted DNA fragments were ligated with *Eco*RI-cleaved and dephosphorylated λZipLox vector arms (Life Technologies, Gaithersburg, MD), and the ligation mixtures were packaged using commercial packaging extracts (Stratagene, La Jolla, CA). The packaged DNA libraries were plated and amplified in *E*. *coli* Y1090ZL cells.

The cDNA from *Fusarium venenatum* clone FA0726 was excised from the vector plasmid by digestion with *Eco*RI and *Not*l yielding an approximately 900 bp fragment. The fragment was purified by gel electrophoresis, and radiolabeled with α[³²P] dCTP using a Prime-it Random Primer Labeling Kit (Stratagene, La Jolla, CA).

Approximately 40,000 plaques from the library were screened by plaque-hybridization (Davis *et al.,* 1980, *supra*) with the radiolabeled probe fragment of the *Fusarium venenatum* lipase gene using high stringency conditions at 45°C (high stringency = 50% formamide, 5X SSPE, 0.3% SDS, 200 µg/ml sheared and denatured salmon sperm DNA). Plaques, which gave hybridization signals, were purified once in *E*. *coli* DH10B cells, and the individual clones were subsequently excised from the λZipLox vector as pZL1-derivatives (D'Alessio et al., 1992, Focus® 14: 7).

One plaque was identified that hybridized strongly to the *Fusarium venenatum* lipase gene probe, and was subsequently excised from the λZipLox vector as a pZL1-derivative (D'Alessio *et al.,* 1992, *supra*). Plasmid DNA was isolated from the clone by passage through *E. coli* DH10B cells (Life Technologies, Gaithersburg, MD) according to the manufacturer's instructions. This clone was designated *E. coli* DH10B - pFvLipase1.

### Example 8: Characterization of the Fusarium venenatum genomic clone encoding lipase

DNA sequencing was performed on an Perkin-Elmer Biosystems Model 377 XL Automated DNA Sequencer using dye-terminator chemistry (Giesecke et al., 1992, Journal of Virology Methods 38: 47-60). Contig sequences were generated using a transposon insertion strategy (Primer Island Transposition Kit, Perkin-Elmer/Applied Biosystems, Inc., Foster City, CA). The 2.94 kb genomic fragment was sequenced to an average redundancy of 4.8.

The nucleotide sequence and deduced amino acid sequence are shown as SEQ ID NO: 1-2. The insert contains an open reading frame of 1.153 kb encoding a polypeptide of 350 amino acids. Using the SignalP software program (Nielsen et al., 1997, Protein Engineering 10: 1-6), a signal peptide of 15 residues was predicted. The predicted signal peptide is followed by a 15 residue propeptide ending with a concanical propeptide Glu/Arg cleavage site. N-terminal sequencing of the lipase protein supports this propeptide cleavage site prediction. The open reading frame is interrupted by two introns of 49 bp and 58 bp. Thus, the mature *Fusarium venenatum* lipase comprises 319 amino acids and a predicted molecular weight of 33.6 kDa. There are 2 potential N-linked glycosylation sites (Asn-X-Ser/Thr) within the *Fusarium venenatum* lipase.

A comparative alignment of lipase sequences using the Clustal W algorithm in the Megalign program of DNA-Star, showed that the deduced amino acid sequence of the *Fusarium venenatum* lipase gene shares 81 % identity to the deduced amino acid sequence of a *Fusarium oxysporum* phospholipase A (EP0869167).

### Example 9: Construction of plasmid pSheB1

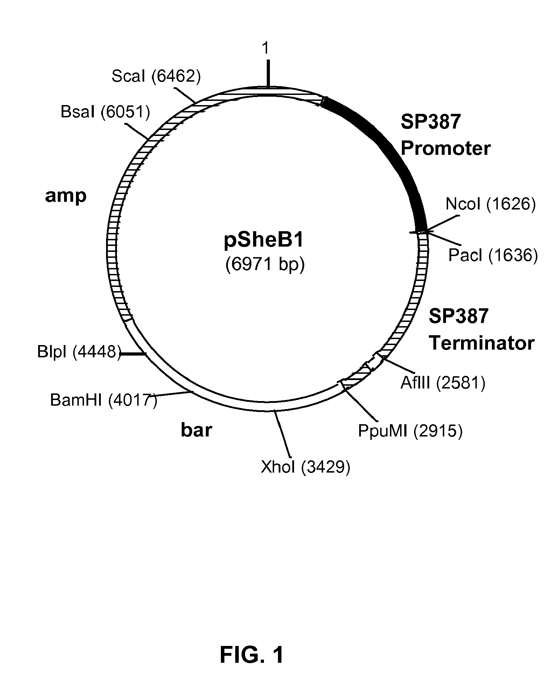

The *Fusarium venenatum* expression vector pSheB1 (Figure 1) was generated by modification of pDM181 (WO 98/20136). The modifications included (a) removal of two Ncol sites within the pDM181 sequence, and (b) restoration of the natural translation start of the *Fusarium oxysporum* trypsin promoter (reconstruction of an Ncol site at the ATG start codon).

Removal of two Ncol sites within the pDM181 sequence was accomplished using the QuikChange™ site-directed mutagenesis kit (Stratagene Cloning Systems, La Jolla, CA) according to the manufacturer's instruction with the following pairs of mutagenesis primers: SEQ ID NO: 11 and 12, SEQ ID NO: 13 and 14.

Restoration of the natural translation start of the *Fusarium oxysporum* trypsin promoter was also accomplished using the Stratagene QuikChange™ site directed mutagenesis kit in conjunction with the following pair of mutagenesis primers: SEQ ID NO:15 and 16.

All site-directed changes were confirmed by DNA sequence analysis of the appropriate vector regions.

### Example 10: Construction of expression vector pEJG60

The lipase-expression vector, pEJG60 was constructed as follows. The lipase coding region was amplified from pFvlipase1 using the following pair of primers: Primer 990658 and 990661 (SEQ ID NO: 17 and 18)

The forward primer introduces a *Sph*l site which contains the ATG, and the reverse primer introduces a *Pac*l site after the stop codon.

The amplification reaction (100 µl) contained the following components: 0.5 µg of genomic clone pFvLipase1, 50 pmol of the forward primer, 50 pmol of the reverse primer, 10 mM dNTPs (dATP, dCTP, dGTP, and dTTP), 1X *Pwo* DNA polymerase buffer, and 2.5 units of *Pwo* DNA polymerase (Boehringer Mannheim, Indianapolis, IN). The reactions were incubated in a Perkin-Elmer Model 480 Thermal Cycler programmed for 1 cycles at 95°C for 2 minutes; 10 cycles at 94°C for 45 seconds, 55°C for 45 seconds, and 72°C for 2 minutes; 17 cycles at 94°C for 45 seconds, 55°C for 45 seconds, and 72°C for 2 minutes with an extension of 20 seconds per cycle; 1 cycle at 72°C for 10 minutes; and a soak cycle at 4°C. The reaction products were isolated on a 1% agarose gel where a 1.15 kb product band was excised from the gel and purified using Qiaquik Gel Extraction Kit (Qiagen, Chatsworth, CA) according to the manufacturer's instructions.

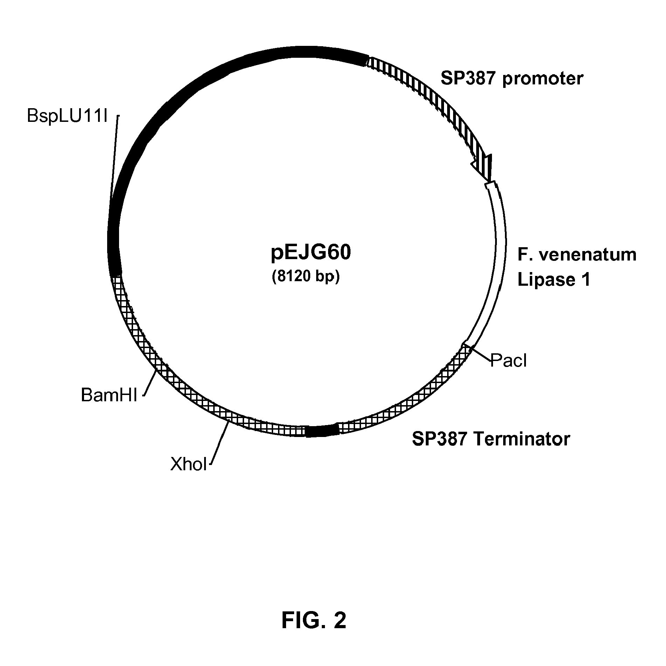

The generated fragment was digested with *Sph*l, blunted with Klenow, digested with *Pac*l, and purified by agarose gel electrophoresis and Qiaquik Gel Extraction Kit (Qiagen, Chatsworth, CA). The purified DNA segment was ligated into pSheB1 (Figure 1) which was previously Ncol digested, treated with DNA polymerase I (Klenow fragment), and digested with *Pac*l. The treatment of the *Nco*l-digested vector with Klenow fragment resulted in a filling in of the Ncol cohesive end, thereby making it blunt and compatible with the blunt site of the lipase DNA segment. The resulting expression plasmid was designated pEJG60 (Figure 2). The PCR-amplified lipase gene segment was re-sequenced to verify the absence of any errors.

### Example 11: Transformation of Fusarium venenatum and analysis of Fusariumvenenatum transformants

Spores of *Fusarium venenatum* WTY700 were generated by inoculating a flask containing 500 ml of RA sporulation medium with 10 plugs from a 1X Vogels medium plate (2.5% Noble agar) supplemented with 2.5% glucose and 2.5 mM sodium nitrate and incubating at 28°C, 150 rpm for 2 to 3 days. Spores were harvested through Miracloth (Calbiochem, San Diego, CA) and centrifuged 20 minutes at 7000 rpm in a Sorvall RC-5B centrifuge (E. I. DuPont De Nemours and Co., Wilmington, DE). Pelleted spores were washed twice with sterile distilled water, resuspended in a small volume of water, and then counted using a hemocytometer.

Protoplasts were prepared by inoculating 100 ml of YEPG medium with 4 X 10⁷ spores of *Fusarium venenatum* WTY700 and incubating for 16 hours at 24°C and 150 rpm. The culture was centrifuged for 7 minutes at 3500 rpm in a Sorvall RT 6000D (E. I. DuPont De Nemours and Co., Wilmington, DE). Pellets were washed twice with 30 ml of 1 M MgSO₄ and resuspended in 15 ml of 5 mg/ml of NOVOZYME 234™ (batch PPM 4356, Novo Nordisk A/S, Bagsværd, Denmark) in 1 M MgSO₄. Cultures were incubated at 24°C and 150 rpm until protoplasts formed. A volume of 35 ml of 2 M sorbitol was added to the protoplast digest and the mixture was centrifuged at 2500 rpm for 10 minutes. The pellet was resuspended, washed twice with STC, and centrifuged at 2000 rpm for 10 minutes to pellet the protoplasts. Protoplasts were counted with a hemocytometer and resuspended in an 8:2:0.1 solution of STC:SPTC:DMSO to a final concentration of 1.25 x 10⁷ protoplasts/ml. The protoplasts were stored at -80°C, after controlled-rate freezing in a Nalgene Cryo 1°C Freezing Container (VWR Scientific, Inc., San Francisco, CA).

Frozen protoplasts of *Fusarium venenatum* WTY700 were thawed on ice. Five µg of pEJG60 described in Example 10 and 5 µl of heparin (5 mg per ml of STC) was added to a 50 ml sterile polypropylene tube. One hundred µl of protoplasts was added, mixed gently, and incubated on ice for 30 minutes. One ml of SPTC was added and incubated 20 minutes at room temperature. After the addition of 25 ml of 40°C COVE top agarose, the mixture was poured onto an empty 150 mm diameter plate and incubated overnight at room temperature. Then an additional 25 ml of 40°C COVE top agarose containing 10 mg of BASTA™ per ml was poured on top of the plate and incubated at room temperature for up to 14 days. The active ingredient in the herbicide BASTA™ is phosphinothricin. BASTA™ was obtained from AgrEvo (Hoechst Schering, Rodovre, Denmark) and was extracted twice with phenol:chloroform:isoamyl alcohol (25:24:1), and once with chloroform:isoamyl alcohol (24:1) before use.

Twenty-four transformants were picked directly from the selection plates (COVE underlay with COVE-BASTA™ overlay) and inoculated into 125 ml shake flasks containing 25 ml of M400Da medium supplemented with 1 mM CaCl₂ and 100 µg/ml ampicillin (to prevent bacterial contamination) and incubated at 28°C, 200 rpm on a platform shaker for 7 days. The untransformed recipient strain was also included as a negative control.

Flasks were sampled at 5 and 7 days and assayed for lipase activity as described below. The samples were also submitted to SDS-PAGE using Novex gradient gels (Novex Experimental Technology, San Diego, CA).

Lipase activity was determined as follows: 100 µl of substrate (3.92 ml of 100 mM MOPS pH 7.5, 4 mM CaCl₂, 990 µl of DMSO, 80 µl of 1% AOS, and 20 µl of p-nitrophenyl butyrate) was added to 100 µl of diluted sample. The samples were diluted accordingly in 100 mM MOPS pH 7.5, 4 mM CaCl₂. The absorbance at 405 nm was monitored for 3 minutes at room temperature in a 96-well microtiter plate using a Molecular Devices Thermomax Microplate Reader.

The lipase assay results indicated that at both 5 and 7 days, most of the transformants produced lipase activity well above that of the untransformed control. Shake flask culture broths from transformants #1 and #3, the two highest scorers in the lipase assay, were analyzed on a 16% tricine gel. A prominent polypeptide at a apparent molecular weight of 32-33 kD was observed at both time points and for each transformant harboring pEJG60.

### Example 12: Cloning and expression of lipase gene from Fusarium sulphureum

### Transformation in Aspergillus strain

*Aspergillus oryzae* strain BECh-2 was inoculated to 100 ml of YPG medium and incubated for16 hrs at 32°C at 120 rpm. Pellets were collected and washed with 0.6 M KCI, and resuspended 20 ml 0.6 M KCI containing a commercial β-glucanase product (Glucanex, product of Novo Nordisk A/S) at the concentration of 30 µl/ml. Cultures were incubated at 32°C at 60 rpm until protoplasts formed, then washed with STC buffer twice. The protoplasts were counted with a hematometer and resuspended in an 8:2:0.1 solution of STC:STPC:DMSO to a final concentration of 2.5x10e7 protoplasts/ml. About 3 µg of DNA was added to 100 µl of protoplasts solution, mixed gently and incubated on ice for 30 min. One ml of SPTC was added and incubated 30 min at 37°C. After the addition of 10 ml of 50°C Cove top agarose, the reaction was poured onto Cove agar plate. Transformation plates were incubated at 32°C for 5 days.

### PCR screening of lipase

A strain of *Fusarium sulphureum* was used as a genomic DNA supplier.

PCR reactions on *Fusarium sulphureum* genomic DNA was done with two following primer sets: lip3 / lip15 (SEQ ID NO: 20/23) and lip10 / lip17 (SEQ ID NO: 21/24) designed based upon the alignment of 3 lipases from *Fusarium.*

Reaction components (2.6 ng /µl of genomic DNA, 250 mM dNTP each, primer 250 nM each, 0.1 U/ µl of Taq polymerase in 1X buffer (Roche Diagnostics, Japan)) were mixed and submitted for PCR under the following conditions.

| Step | Temperature | Time |
|---|---|---|
| 1 | 94°C | 1 min |
| 2 | 50°C | 1 min |
| 3 | 72°C | 2 min |
| 4 | 72°C | 10 min |
| 5 | 4°C | forever |

Steps 1 to 3 were repeated 30 times.

450bp of fragment and 280 bp of fragment were amplified from primer sets of lip3/1ip15 (SEQ ID NO: 20/23) and Iip10/1ip17 (SEQ ID NO: 21/24), respectively. They were gel-purified with GFX^{™} PCR DNA and Gel Band Purification kit (amersham pharmacia biotech) and ligated into a pT7Blue vector with ligation high (TOYOBO, Japan) . The ligation mixtures were transformed into *E. coli* JM109. The resultant plasmids, pT27-0315 and pT27-1017, were sequenced and compared to the *Fusarium oxysporum* lipase, showing that a clone encodes the internal part of the lipase.

### Cloninq of lipase qene

In order to clone the missing part of the lipase gene, adaptor PCR was done. A cassette was made by mixing of adaptor L (SEQ ID NO: 26) and adaptor S:: acctgccc.

3' and 5' of adaptor S are dephosphorylated and amidated, respectively.

1.3 µg of Eco RV digested genome was ligated to the cassette and it was used as a PCR template. Reaction components (7 ng /µl of genomic DNA ligated to cassette, 250 mM dNTP each, primer 250 nM each, 0.05 U/ µl of Expand high fidelity polymerase in 1X buffer (Roche Diagnostics, Japan)) were mixed and submitted for PCR under the following conditions.

| Step | Temperature | Time |
|---|---|---|
| 1 | 94°C | 2 min |
| 2 | 94°C | 10sec |
| 3 | 55°C | 30sec |
| 4 | 68°C | 45sec |

| step 2-4 repeat 10 times | | |
|---|---|---|
| 5 | 94°C | 10sec |
| 6 | 55°C | 30sec |
| 7 | 68°C | 45sec |
| | | +20sec/cycle |

| step 5-7, repeat 20 times | | |
|---|---|---|
| 8 | 68°C | 7min |
| 7 | 4°C | forever |

500 bp of DNA fragment corresponding to N-terminal region was obtained with 27N1long primer (SEQ ID NO: 27) and 200 bp of DNA fragment corresponding to C-terminal region was obtained with 27C1long primer (SEQ ID NO: 28).

Obtained fragments were purified by GFX^{™} PCR DNA and Gel Band Purification kit (Amersham Pharmacia Biotech) and sequenced with each primers which amplified the fragment.

The missing C-terminal part was cloned with LA PCR^{™} in vitro Cloning Kit (TaKaRa) following to the manufacturer's instructions. 350 bp of DNA fragment corresponding to C-terminal region was obtained from Xho I digested genome ligated to Sal I cassette of the kit with 27C2 primer (SEQ ID NO: 29).

Obtained fragments were purified by GFX^{™} PCR DNA and Gel Band Purification kit (Amersham Pharmacia Biotech) and sequenced with 27C2 primer.

The fidelity of taq polymerase is not good so in order to get the right sequence whole gene was amplified the following primers: 27N(Bam) and 27C(Sal) (SEQ ID NO: 30 and 31).

Reaction components (6 ng /µl of genomic DNA, 250 mM dNTP each, primer 250 nM each, 0.05 U/ µl of Expand high fidelity polymerase in 1X buffer (Roche Diagnostics, Japan)) were mixed and submitted for PCR under the following conditions.

| Step | Temperature | Time |
|---|---|---|
| 1 | 94°C | 2 min |
| 2 | 94°C | 10sec |
| 3 | 55°C | 30sec |
| 4 | 68°C | 45sec |

| step 2-4 repeat 10 times | | |
|---|---|---|
| 5 | 94°C | 10sec |
| 6 | 55°C | 30sec |
| 7 | 68°C | 45sec |
| | | +20sec/cycle |

| step 5-7, repeat 20 times | | |
|---|---|---|
| 8 | 68°C | 7min |
| 7 | 4°C | forever |

Amplified DNA fragment was gel-purified with GFX^{™} PCR DNA and Gel Band Purification kit (amersham pharmacia biotech) and ligated into a pT7Blue vector with ligation high (TOYOBO, Japan). The ligation mixtures were transformed into *E. coli* JM1 09. Four plasmids, pT27w-1, pT27w-2, pT27w-3, and pT27w-4, were sequenced and their sequence were compared. pT27w-3 has no PCR error and it is defined as *Fusarium sulphureum* lipase nucleotide sequence.

### Expression of lipase gene in Aspergillus oryzae.

The lipase gene was digested from pT27w-3 with BamH I and Sal I and ligated into the BamH I and Xhol sites in the *Aspergillus* expression cassette pCaHj483 which has *Aspergillus niger* neutral amylase promoter, *Aspergillus nidulans* TPI leader sequences, *Aspergillus niger* glucoamylase terminator and *Aspergillus nidulans* amdS gene as a marker. The resultant plasmid was pNL27w-8.

pNL27w-8 was transformed into *Aspergillus oryzae* BECh-2. The selected transformants were inoculated in 100 ml of MS-9 media and cultivated at 30°C for 1 day. 3 ml of grown cell in MS-9 medium was inoculated to 100 ml of MDU-2BP medium and cultivated at 32°C for 3 days. The supernatant was obtained by centrifugation.

The lipase productivity of selected transformants was determined as LU activity. The productivity of the best transformant TNL27-75 was 130 LU/ml and BECh2 has no lipase activity.

### Example 13: Immunological characterization of lipolytic enzyme from Fusarium sulphureum

A purified lipolytic enzyme sample having the amino acid sequence shown as amino acids 1-319 of SEQ ID NO: 4 was tested by immunodiffusion against a polyclonal antibody raised against the *Fusarium oxysporum* lipase. No immunological cross-reaction was found.

### Example 14: Cloning and expression of lipase gene from Acremonium berkeleyanum

### Transformation in Aspergillus strain

*Aspergillus oryzae* strain BECh-2 was inoculated to 100 ml of YPG medium and incubated for16 hrs at 32°C at 120 rpm. Pellets were collected and washed with 0.6 M KCI, and resuspended 20 ml 0.6 M KCI containing a commercial β-glucanase product (Glucanex, product of Novo Nordisk A/S) at the concentration of 30 µ/ml. Cultures were incubated at 32°C at 60 rpm until protoplasts formed, then washed with STC buffer twice. The protoplasts were counted with a hematometer and resuspended in an 8:2:0.1 solution of STC:STPC:DMSO to a final concentration of 2.5x10e7 protoplasts/ml. About 3 µg of DNA was added to 100 µl of protoplasts solution, mixed gently and incubated on ice for 30 min. One ml of SPTC was added and incubated 30 min at 37°C. After the addition of 10 ml of 50°C Cove top agarose, the reaction was poured onto Cove agar plate. Transformation plates were incubated at 32°C for 5 days.

### PCR screening of lipase

A strain of *Acremonium berkeleyanum* was used as a genomic DNA supplier.

PCR reactions on *Acremonium berkeleyanum* genomic DNA was done with two following primer sets: lip3 / lip11 (SEQ ID NO: 20/22) and Iip10 / lip21 (SEQ ID NO: 21/25) designed based upon the alignment 3 lipases from *Fusarium.*

Reaction components (2.5 ng /µl of genomic DNA, 250 mM dNTP each, primer 250 nM each, 0.1 U/ µl in Taq polymerase in 1X buffer (Roche Diagnostics, Japan)) were mixed and submitted for PCR under the following conditions.

| Step | Temperature | Time |
|---|---|---|
| 1 | 94°C | 1 min |
| 2 | 50°C | 1 min |
| 3 | 72°C | 2 min |
| 4 | 72°C | 10 min |
| 5 | 4°C | forever |

Steps 1 to 3 were repeated 30 times.

340 bp of fragment and 330 bp of fragment were amplified from primer sets of lip3/lip11 and lip10/lip21, respectively. They were gel-purified with GFX^{™} PCR DNA and Gel Band Purification kit (amersham pharmacia biotech) and ligated into a pT7Blue vector with ligation high (TOYOBO, Japan) . The ligation mixtures were transformed into *E*. *coli* JM109. The resultant plasmids, pTAc-0310 and pTAc-1021, were sequenced and compared to the *Fusarium oxysporum* lipase, showing that a clone encodes the internal part of the lipase.

### Cloninq of lipase gene

In order to clone the missing part of the lipase gene, LA PCR^{™} in vitro Cloning Kit (TaKaRa) was used for genome walking. One kb of DNA fragment corresponding to N-terimal region was obtained from Sal I digested genome ligated to Sal I cassette of the kit with AcN3 primer (SEQ ID NO: 32). One kb of DNA fragment corresponding to C-terminal region was obtained from Hind III digested genome ligated to Hind III cassette of the kit with AcC3 primer (SEQ ID NO: 33).

Obtained fragments were purified by GFX^{™} PCR DNA and Gel Band Purification kit (amersham pharmacia biotech) and sequenced with each primer which amplified the fragment. The sequences were compared to *Fusarium oxysporum* lipase, showing that an amplified DNA fragments encodes the whole part of lipase.

The fidelity of taq polymerase is not good so in order to get the right sequence whole gene was amplified the primers 152-N (Bcl) and 152-C(Xho) (SEQ ID NO: 34 and 35).

Reaction components (6 ng /µl of genomic DNA, 250 mM dNTP each, primer 250 nM each, 0.05 U/ µl of Expand high fidelity polymerase in 1X buffer (Roche Diagnostics, Japan)) were mixed and submitted for PCR under the following conditions.

| Step | Temperature | Time |
|---|---|---|
| 1 | 94°C | 2 min |
| 2 | 94°C | 10sec |
| 3 | 55°C | 30sec |
| 4 | 68°C | 45sec |

| step 2-4 repeat 10 times | | |
|---|---|---|
| 5 | 94°C | 10sec |
| 6 | 55°C | 30sec |
| 7 | 68°C | 45sec |
| | | +20sec/cycle |

| step 5-7, repeat 20 times | | |
|---|---|---|
| 8 | 68°C | 7min |
| 7 | 4°C | forever |

Amplified DNA fragment was gel-purified with GFX^{™} PCR DNA and Gel Band Purification kit (amersham pharmacia biotech) and ligated into a pT7Blue vector with ligation high (TOYOBO, Japan). The ligation mixtures were transformed into *E. coli* JM1 09. The resultant plasmids, pT152-1 and pT152-2, were sequenced and they are identical. pT152-1 sequence is defined as *Acremonium berkeleyanum* lipase nucleotide sequence.

### Construction of expression plasmid pMT 2188.

*Aspergillus oryzae* expression plasmid pCaHj 483 (described in WO 98/00529) consists of an expression cassette based on the *Aspergillus niger* neutral amylase II promoter fused to the *Aspergillus nidulans* triose phosphate isomerase non translated leader sequence (Pna2/tpi) and the *Aspergillus niger* amyloglycosidase terminater (Tamg). Also present on the plasmid is the *Aspergillus* selective marker *amdS* from *Aspergillus nidulans* enabling growth on acetamide as sole nitrogen source. These elements were cloned into the *E. coli* vector pUC19. The ampicillin resistance marker enabling selection in *E*. *coli* of this plasmid was replaced with the URA3 marker of *Saccharomyces cerevisiae* that can complement a *pyrF* mutation in *E. coli* in the following way:
The pUC19 origin of replication was PCR amplified from pCaHj483 with the primers 142779 and 142780 (SEQ ID NO: 40 and 41). The primer 142780 introduces a Bbu I site in the PCR fragment.
The Expand PCR system (Roche Molecular Biochemicals, Basel, Switserland) was used for the amplification following the manufacturers instructions for this and the subsequent PCR amplifications.
The URA3 gene was amplified from the general *S. cerevisiae* cloning vector pYES2 (Invitrogen corporation, Carlsbad, Ca, USA) using the primers 140288 and 142778 (SEQ ID NO: 36 and 39). The primer 140288 introduces an EcoR I site in the PCR fragment.
The two PCR fragments were fused by mixing them and amplifying using the primers 142780 and 140288 using the splicing by overlap method (Horton et al (1989) Gene, 77, 61-68).
The resulting fragment was digested with EcoR I and Bbu I and ligated to the largest fragment of pCaHj 483 digested with the same enzymes. The ligation mixture was used to transform the *pyrF⁻ E. coli* strain DB6507 (ATCC 35673) made competent by the method of Mandel and Higa (Mandel, M. and A. Higa (1970) J. Mol. Biol. 45, 154). Transformants were selected on solid M9 medium (Sambrook et. al (1989) Molecular cloning, a laboratory manual, 2. edition, Cold Spring Harbor Laboratory Press) supplemented with 1 g/I casaminoacids, 500 µg/l thiamine and 10 mg/l kanamycin.
A plasmid from such a transformant was termed pCaHj 527.
ThePna2/tpi promoter present on pCaHj527 was subjected to site directed mutagenises by a simple PCR approach.
Nucleotide 134 - 144 was altered from gtactaaaacc (SEQ ID NO: 57) to ccgttaaattt (SEQ ID NO: 58) using the mutagenic primer 141223 (SEQ ID NO: 38).
Nucleotide 423 - 436 was altered from atgcaatttaaact (SEQ ID NO: 59) to cggcaatttaacgg (SEQ ID NO: 60) using the mutagenic primer 141222: (SEQ ID NO: 37). The resulting plasmid was termed pMT 2188.

### Expression of lipase gene in Aspergillus oryzae.

The plasmid pT152-1 was transformed to JM110 and non-methylated pT152-1 was extracted. The lipase gene was digested from non-methylated pT152-1 with Bcl I and Xho I and ligated into the BamH I and Xhol sites in the *Aspergillus* expression cassette pMT2188 which has *Aspergillus niger* neutral amylase promoter, *Aspergillus nidulans* TPI leader sequences, *Aspergillus niger* glucoamylase terminator and *Aspergillus nidulans* amdS gene as a marker and Saccharomyces cerevisiae URA3 gene as a marker for a plasmid construction. The ligation mixture was transformed E.coli 6507 by electroporation and the resultant plasmid was pNL152-3 (24).

pNL152-3 (24) was transformed into *Aspergillus oryzae* BECh-2. The selected transformants were inoculated in 100 ml of MS-9 media and cultivated at 30°C for 1 day. 3 ml of grown cell in MS-9 medium was inoculated to 100 ml of MDU-2BP medium and cultivated at 32°C for 3 days. The supernatant was obtained by centrifugation.

The lipase productivity of selected transformants was determined as LU activity.

### Example 15: Cloning and expression of lipase gene from Fusarium culmorum

### Transformation in Aspergillus strain

*Aspergillus oryzae* strain BECh-2 was inoculated to 100 ml of YPG medium and incubated for16 hrs at 32°C at 120 rpm. Pellets were collected and washed with 0.6 M KCI, and resuspended 20 ml 0.6 M KCI containing a commercial β-glucanase product (Glucanex, product of Novo Nordisk A/S) at the concentration of 30 µl/ml. Cultures were incubated at 32°C at 60 rpm until protoplasts formed, then washed with STC buffer twice. The protoplasts were counted with a hematometer and resuspended in an 8:2:0.1 solution of STC:STPC:DMSO to a final concentration of 2.5x10e7 protoplasts/ml. About 3 µg of DNA was added to 100 µl of protoplasts solution, mixed gently and incubated on ice for 30 min. One ml of SPTC was added and incubated 30 min at 37°C. After the addition of 10 ml of 50°C Cove top agarose, the reaction was poured onto Cove agar plate. Transformation plates were incubated at 32°C for 5 days.

### PCR screening of lipase

A strain of *Fusarium culmorum* was used as a genomic DNA supplier.

PCR reactions on *Fusarium culmorum* genomic DNA was done with two following primer set: lip2 / lip21 (SEQ ID NO: 19/25) designed based upon the alignment 3 lipases from *Fusarium.*

Reaction components (6 ng /µl of genomic DNA, 250 mM dNTP each, primer 250 nM each, 0.1 U/ µl in Taq polymerase in 1X buffer (Roche Diagnostics, Japan)) were mixed and submitted for PCR under the following conditions.

| Step | Temperature | Time |
|---|---|---|
| 1 | 94°C | 1 min |
| 2 | 50°C | 1 min |
| 3 | 72°C | 2 min |
| 4 | 72°C | 10 min |
| 5 | 4°C | forever |

Steps 1 to 3 were repeated 30 times.

0.7 kbp of fragment was amplified. It was gel-purified with GFX^{™} PCR DNA and Gel Band Purification kit (Amersham Pharmacia Biotech) and ligated into a pT7Blue vector with ligation high (TOYOBO, Japan) . The ligation mixtures were transformed into *E. coli* JM1 09. The resultant plasmids, pT12-0221 was sequenced and compared to the *Fusarium oxysporum* lipase, showing that a clone encodes the internal part of the lipase.

### Cloninq of lipase gene

In order to clone the missing part of the lipase gene, LA PCR^{™} in vitro Cloning Kit (TaKaRa) was used for genome walking. 0.5 kbp of DNA fragment corresponding to N-terimal region was obtained from BamH I digested genome ligated to Sau3A I cassette of the kit with 12N1 primer (SEQ ID NO: 42). 1.8 kb of DNA fragment corresponding to C-terminal region was obtained from Bgl II digested genome ligated to Sau3A I cassette of the kit with 12C2 primer (SEQ ID NO: 43).

Obtained fragments were purified by GFX^{™} PCR DNA and Gel Band Purification kit (amersham pharmacia biotech) and sequenced with each primer which amplified the fragment. Their sequence were compared to the Fusarium oxysporum lipase, showing that the amplified DNA covered N-terminal and C-terminasl part of the lipase.

The fidelity of taq polymerase is not so good so in order to get the right sequence whole gene was amplified the primers 12-N (Bcl) and 12-C(Sal) (SEQ ID NO: 44 and 45).

Reaction components (6 ng /µl of genomic DNA, 250 mM dNTP each, primer 250 nM each, 0.05 U/ µl of Expand high fidelity polymerase in 1X buffer (Roche Diagnostics, Japan) were mixed and submitted for PCR under the following conditions.

| Step | Temperature | Time |
|---|---|---|
| 1 | 94°C | 2 min |
| 2 | 94°C | 10sec |
| 3 | 55°C | 30sec |
| 4 | 68°C | 45sec |

| step 2-4 repeat 10 times | | |
|---|---|---|
| 5 | 94°C | 10sec |
| 6 | 55°C | 30sec |
| 7 | 68°C | 45sec |
| | | +20sec/cycle |

| step 5-7, repeat 20 times | | |
|---|---|---|
| 8 | 68°C | 7min |
| 7 | 4°C | forever |

An amplified DNA fragment was gel-purified with GFX^{™} PCR DNA and Gel Band Purification kit (amersham pharmacia biotech) and ligated into a pT7Blue vector with ligation high (TOYOBO, Japan). The ligation mixtures were transformed into *E. coli* JM109. The resultant plasmids, pT12-1, pT12-2, pT12-3, and pT12-4, were sequenced and all of them are identical. The sequence is defined as *Fusarium culmorum* lipase DNA sequence.

### Expression of lipase gene in Aspergillus oryzae.

The plasmid pT12-6 was transformed to JM110 and non-methylated pT12-6 was extracted. The lipase gene was digested from non-methylated pT12-6 with Bcl I and Sal I into T-vector and ligated into the BamH I and Xhol sites in the *Aspergillus* expression cassette pMT2188 which has *Aspergillus niger* neutral amylase promoter, *Aspergillus nidulans* TPI leader sequences, *Aspergillus niger* glucoamylase terminator and *Aspergillus nidulans* amdS gene as a marker and Saccharomyces cerevisiae URA3 gene as a marker for a plasmid construction. The ligation mixture was transformed E.coli 6507 by electroporation and the resultant plasmid was pNL12-***.

pNL12-*** was transformed into *Aspergillus oryzae* BECh-2. The selected transformants were inoculated in 100 ml of MS-9 media and cultivated at 30°C for 1 day. 3 ml of grown cell in MS-9 medium was inoculated to 100 ml of MDU-2BP medium and cultivated at 32°C for 3 days. The supernatant was obtained by centrifugation.

The lipase productivity of selected transformants was determined as LU activity.

### Example 16: Cloning of a phospholipase gene from the Fusarium solani strain MUCL 38667.

A genomic DNA preparation of the strain MUCL 38667 was made as described in WO 00/24883.

A PCR reaction (96°C 5 min, 30* (94°C 30 sec., 55°C 30 sec, 72°C 1 min), 72°C 5 min) was run using PWO polymerase in 2.5 mM MgSO₄ as recommended by the manufacturer (Boehringer Mannheim) with the MUCL 38667 genomic DNA as template, with oligo 161000J1 and 161000J2 (SEQ ID NO: 46 and 47). These oligo'es were designed based conserved sequences in homologous phospholipases.

A fragment of 180 bp was isolated from a 2% gel. Because the amounts of DNA was very small, a new identical pcr was run, this time using the 180 bp fragment as template rather than MUCL 38667 genomic DNA.

This fragment was cloned into pCR4 using TOPO-cloning as recommended by the manufacture (Invitrogen) and transformed into the *E. coli* strain TOPO10.

DNA preparations where made using the Qiagen minispinprep kit and the clones where sequenced using M13 rev and M13 fwp primer supplied with the TOPO-cloning kit (Invitrogen). Sequence alignment was made to all available DNA sequence using SRS. The 180 bp fragment was identified as originating from a phospholipase gene.

Based on the 180 bp fragment DNA sequence, four primers were designed: 071200J1, 071200j2, 221200J1, 221200J2 (SEQ ID NO: 48-51).

The MUCL38667 genomic DNA (app. 1 µg) was cut with Agel in a volume of 10 µl and ligated in a volume of 500 µl. The DNA was precipitated in ethanol and redisolved in water. 2 µl of the religated mix was used as template, and oligo 071200J1 and 071200J2 in a PCR reaction using GeneAMP XL PCR kit as recommended by manufacture (Boehringer Mannheim) in a total of 20 µl.

1 µl of this PCR reaction fragments was used as template in a second PCR reaction using nested oligoes 221200J1 and 221200J2 (SEQ ID NO: 50 and 51), which was identical to the above mentioned.

The generated PCR fragment of app. 1500 bp was cloned into pCR4 using TOPO-cloning as recommended by the manufacture (Invitrogen) and transformed into the *E*. *coli* strain TOPO10.

DNA preparations where made using the Qiagen minispinprep kit and the clones where sequenced using M13 rev and M13 fwp primer supplied with the TOPO-cloning kit (Invitrogen). Sequence alignment was made to all available DNA sequence using SRS. The 1500 bp fragment was identified as originating from the 3' end of a phospholipase gene.

Based on the 1500 bp fragment DNA sequence, one primer was designed: 170101J11 (SEQ ID NO: 52).

The MUCL 38667 genomic DNA (app. 1 µg) was cut with HindIII in a volume of 10 µl and ligated in a volume of 500 µl. The DNA was precipitated in ethanol and redisolved in water. 2 µl of the religated mix was used as template, and oligo 221200J1 and 170101J11 in a PCR reaction using GeneAMP XL PCR kit as recommended by manufacture (Boehringer Mannheim) in a total of 20 µl.

The generated PCR fragment of app. 350 bp was cloned into pCR4 using TOPO-cloning as recommended by the manufacture (Invitrogen) and transformed into the *E*. *coli* strain TOPO10.

DNA preparations were made using the Qiagen minispinprep kit and the clones where sequenced using T3 and T7 primer supplied with the TOPO-cloning kit (Invitrogen). Sequence alignment was made to all available DNA sequence using SRS. The 350 bp fragment was identified as originating from the 5 'end of a phospholipase gene.

Based on the 350 bp and the 1500 bp DNA sequence, two primers were designed (290101j2 and 020301j1, SEQ ID NO: 53 and 54), thus covering the hole gene.

A PCR reaction (96°C 5 min, 30* (94°C 30 sec., 55°C 30 sec, 72°C 2 min), 72°C 5 min) was run using PWO polymerase in 2.5 mM MgSO₄ as recommended by manufacture (Boehringer Mannheim) with the MUCL38667 genomic DNA as template, with oligo 290101J2 and 020301J1 (SEQ ID NO: 53 and 54).

The generated PCR fragment of app. 1100 bp were cloned into pCR4 using TOPO-cloning as recommended by the manufacture (Invitrogen) and transformed into the *E*. *coli* strain TOPO10.

DNA preparations were made using the Qiagen minispinprep kit and the clones where sequenced using T3 and T7 primer supplied with the TOPO-cloning kit (Invitrogen). Sequence alignment was made to all available DNA sequence using SRS, as well as earlier sequence of the same.

### Example 17: Cloning and expression of lipase gene from Fusarium solani

The cloned phospholipase gene in the TOPO vector, as well as pJVi9 (WO 97/47746) was cut with the restriction enzymes BamHI and Xhol. The pJVI9 vector and the phospholipase gene were purified from a 1% agarose gel, and ligated o/n.

The ligation was transformed into the *E.coli* strain DH10b, and transformants were isolated.

DNA preparations where made using the Qiagen minispinprep kit and the clones where verified by sequencing using 19670 and 19671 primer (SEQ ID NO: 55 and 56).

The resulting plasmid was transformed into the *Aspergillus oryzae* strain Jal125 (WO 97/35956) using the following method:

### Transformation of Aspergillus oryzae (general procedure)

100 ml of YPD (Sherman et al., (1981), Methods in Yeast Genetics, Cold Spring Harbor Laboratory) are inoculated with spores of *A. oryzae* and incubated with shaking for about 24 hours. The mycelium is harvested by filtration through miracloth and washed with 200 ml of 0.6 M MgSO₄. The mycelium is suspended in 15 ml of 1.2 M MgSO₄, 10 mM NaH₂PO₄, pH 5.8. The suspension is cooled on ice and 1 ml of buffer containing 120 mg of Novozym^{®} 234 is added. After 5 min., 1 ml of 12 mg/ml BSA (Sigma type H25) is added and incubation with gentle agitation continued for 1.5-2.5 hours at 37°C until a large number of protoplasts is visible in a sample inspected under the microscope.

The suspension is filtered through miracloth, the filtrate transferred to a sterile tube and overlayed with 5 ml of 0.6 M sorbitol, 100 mM Tris-HCI, pH 7.0. Centrifugation is performed for 15 min. at 1000 g and the protoplasts are collected from the top of the MgSO₄ cushion. 2 volumes of STC (1.2 M sorbitol, 10 mM Tris-HCI, pH 7.5, 10 mM CaCl₂) are added to the protoplast suspension and the mixture is centrifuged for 5 min. at 1000 g. The protoplast pellet is resuspended in 3 ml of STC and repelleted. This is repeated. Finally, the protoplasts are resuspended in 0.2-1 ml of STC.

100 µl of protoplast suspension are mixed with 5-25 µg of p3SR2 (an A. *nidulans* amdS gene carrying plasmid described in Hynes et al., Mol. and Cel. Biol., Vol. 3, No. 8, 1430-1439, Aug. 1983) and 5 µg of the pJV19-phospholipase plasmid in 10 µl of STC. The mixture is left at room temperature for 25 min. 0.2 ml of 60% PEG 4000 (BDH 29576), 10 mM CaCl₂ and 10 mM Tris-HCI, pH 7.5 is added and carefully mixed (twice) and finally 0.85 ml of the same solution are added and carefully mixed. The mixture is left at room temperature for 25 min., spun at 2.500 g for 15 min. and the pellet is resuspended in 2 ml of 1.2M sorbitol. After one more sedimentation the protoplasts are spread on minimal plates (Cove, (1966), Biochem. Biophys. Acta 113, 51-56) containing 1.0 M sucrose, pH 7.0, 10 mM acetamide as nitrogen source and 20 mM CsCl to inhibit background growth. After incubation for 4-7 days at 37°C spores are picked, suspended in sterile water and spread for single colonies.

12 independent transformants from the pJV19-phorpholipase transformations were isolated on minimal plates (Cove, (1966), Biochem. Biophys. Acta 113, 51-56) containing 1.0 M sucrose, pH 7.0, 10 mM acetamide as nitrogen source and 20 mM CsCl to inhibit background growth, and at the same time inoculated into a 96-well microtiter dish containing 200 µl minimal media of 1*vogel, 2% maltose (e.g., Methods in Enzymology, Vol. 17 p. 84) in each well.

After three days of incubation at 34°C, media from the cultures in the microtiter dish were assayed for lipase activity. A 10 µl aliquot of media from each well was added to a microtiter well containing 200 µl of a lipase substrate of 0.018% p-nitrophenylbutyrate, 0.1% Triton X-100, 10 mM CaCl₂, 50 mM Tris pH 7.5. Activity was assayed spectrophotometrically at 15-second intervals over a five minute period, using a kinetic microplate reader (Molecular Device Corp., Sunnyvale CA), using a standard enzymology protocol (e.g., Enzyme Kinetics, Paul C. Engel, ed., 1981, Chapman and Hall Ltd.) Briefly, product formation is measured during the initial rate of substrate turnover and is defined as the slope of the curve calculated from the absorbance at 405 nm every 15 seconds for 5 minutes.

This procedure was repeated and spores of the best producing transformants after the second re-isolation were stored as a defined transformant.

*A. oryzae* JaL 125 (WO 97/35956) is derived from *Aspergillus oryzae* IFO 4177 available from Institute for Fermention, Osaka; 17-25 Juso Hammachi 2-Chome Yodogawa-ku, Osaka, Japan, having the alkaline protease gene named "alp" (described by Murakami K et al., (1991), Agric. Biol. Chem. 55, p. 2807-2811) deleted by a one step gene replacement method (described by G. May in "Applied Molecular Genetics of Filamentous Fungi" (1992), p. 1-25. Eds. J. R. Kinghorn and G. Turner; Blackie Academic and Professional), using the *A. oryzae* pyrG gene as marker.

### Example 18: Preparation and testing of variant

An R275A variant of *Acremonium berkeleyanum* lipase (SEQ ID NO: 6) was constructed and expressed in *A. oryzae.* Preliminary tests using crude culture broth confirmed the variant enzyme to be more heat stable than the wildtype. SDS-PAGE showed that around 60% of the molecules still were processed, compared to 100% for the wildtype. The temperature-stability curve of the crude variant broth was found to be biphasic, and the unprocessed form seemed to be around 10°C more stable than the processed one.

Two bands were seen in SDS-PAGE: unprocessed lipase at around 31 kDa and C-terminal processed at ca. 28 kDa. It was found that unprocessed lipase is stable for 30 minutes up until ca. 60°C, whereas the wildtype, processed lipase is degraded quickly at temperature over 50°C.

The above findings indicate that the R275A variant of *Acremonium berkeleyanum* lipase is to a certain degree protected against KEX2 type protease attack in *Aspergillus oryzae.*

### Example 19: Determination of phospholipase and galactolipase activities

The lipolytic enzymes from *F*. *venenatum, F.sulphureum, A. berkeleyanum, F. culmorum and F. solani* (SEQ ID NO: 2, 4, 6, 8 and 10) were tested for phospholipase and galactolipase activities by plate assay methods, as described in WO 0032758. The lipolytic enzyme from *F*. *oxysporum* was included for comparison.

The results showed that all 5 enzymes have phospholipase activity. The enzymes from *F*. *venenatum, F.sulphureum* and *F*. *culmorum* and *F*. *oxysporum* have activities at a similar level, and the other two have slightly lower activities.

All 5 enzymes were found to have galactolipase activity (digalactosyl diglyceride hydrolyzing activity). The enzymes from *F*. *venenatum, F.sulphureum, F. culmorum* and *F*. *oxysporum* have significantly higher activity than those from *A*. *berkeleyanum* and *F*. *solani.*

### Example 20: Preparation of straight-dough bread

Doughs were made using spiral mixers from 2 kg of Meneba flour (batch 941-2) according to the straight dough method (AACC Method 10-10B in Approved Methods of the American Association of Cereal Chemists, Ninth Edition, March 1995; AACC, St. Paul MN, USA).

Lipase activity was determined using pH-stat titration and tributyrin as substrate at 30°C and pH 7. Enzymes were dosed according to the protocol below:

| Dough | | | | | | | | |
|---|---|---|---|---|---|---|---|---|
| | 1 | 2 | 3 | 4 | 5 | 6 | 7 | 8 |
| Enzyme | | | | | | | | |
| *F. venenatum* Lipase, LU/kg flour | | | 10 | 20 | 40 | 80 | 100 | 150 |
| *F. oxysporum* Lipase, LU/kg flour | | | | | | | | |
| | | 1000 | | | | | | |
| Fungamyl, FAU/kg flour | 10 | 10 | 10 | 10 | 10 | 10 | 10 | 10 |
| PentMono, FXU/kg flour | 100 | 100 | 100 | 100 | 100 | 100 | 100 | 100 |
| DATEM, % | 0.4 | | | | | | | |

### Example 21: Dough and bread evaluation and dough stability measurements

Each dough described in the previous Example was split into 15 rolls for 45 minute fermentation; 15 rolls for 70 minute fermentation. Two pan breads were evaluated for of the breads crumb structure; and 2 pan breads were evaluated for texture analysis.

Firmness, elasticity of the doughs, and crumb structure, texture of the breads, shape factor and volume of the rolls were measured using the methods described earlier. Bread was measured 2 and 24 hours after baking.

The results of Dough 5 showed that the effect of the *Fusarium venenatum* lipase dosed at 40 LU/kg flour was similar to that of the *Fusarium oxysporum* lipase dosed at 1000 LU/kg flour. Increased dosages of the *Fusarium venenatum* lipase resulted in softer, more sticky dough which had a lower extensibility and higher elasticity. At a dosage of 150 LU/kg flour, the *Fusarium venenatum* lipase yielded a dough which broke apart easily when stretched, similar to undermixed doughs.

The effect of the *Fusarium venenatum* lipase on dough parameters was dosage dependent. Generally bread made from dough treated with the *Fusarium venenatum* lipase yielded a more uniform crumb, a coarser grain with thicker cell walls and less elongated shape, and a less white crumb colour compared to *Fusarium oxysporum* lipase.

The effect of the lipases on stability is shown in Tables 1 and 2 at 45 minutes and 70 minutes, respectively. After normal fermentation time, addition of the *Fusarium venenatum* lipase yielded a very good stability factor when dosed at and above 80 LU/kg flour. At dosages of 100 and 150 LU of the *Fusarium venenatum* lipase per kg flour, the volume of the bread matched that of bread treated with DATEM (di-acetylated-tartaric acid-esters of mono-and diglycerides of fatty acids), but was slightly smaller than that of the bread treated with the *Fusarium oxysporum* lipase. The best shape-factors were obtained with the high dosages of the *Fusarium venenatum* lipase.

**Table 1**

| **Lipolytic enzyme added** | **Bread** | |
|---|---|---|
| | **Sp. Vol. (ml/g)** | **Shape factor** |
| | **45 min** | **45 min** |
| **Datem** | 6.89 | 0.672 |
| **F. oxysporum (200LU)** | 7.18 | 0.696 |
| **F. venenatum (10LU)** | 6.34 | 0.663 |
| **F. venenatum (20LU)** | 6.64 | 0.681 |
| **F. venenatum (40LU)** | 6.45 | 0.653 |
| **F. venenatum (80LU)** | 6.51 | 0.687 |
| **F. venenatum (100LU)** | 6.75 | 0.716 |
| **F. venenatum (150LU)** | 6.85 | 0.700 |

**Table 2**

| **Lipolytic enzyme added** | **Bread** | |
|---|---|---|
| | **Sp. Vol. (ml/g)** | **Shape factor** |
| | **70 min** | **70 min** |
| **Datem** | 8.11 | 0.652 |
| **F. oxysporum (200LU)** | 7.96 | 0.682 |
| **F. venenatum (10LU)** | 6.76 | 0.648 |
| **F. venenatum (20LU)** | 6.79 | 0.655 |
| **F. venenatum (40LU)** | 6.94 | 0.651 |
| **F. venenatum (80LU)** | 7.54 | 0.672 |
| **F. venenatum (100LU)** | 7.69 | 0.678 |
| **F. venenatum (150LU)** | 7.78 | 0.680 |

After extended fermentation time, 80 - 150 LU/kg flour of the *Fusarium venenatum* lipase yielded a better shape-factor than DATEM, and 150 LU/kg flour of the *Fusarium venenatum* lipase performed as well as the *Fusarium oxysporum* lipase. A larger volume was achieved with increased dosage of the *Fusarium venenatum* lipase, where at 150 LU of the lipase per kg of flour, the volume increase nearly matched the volume increase with the *Fusarium oxysporum* lipase and DATEM.

When dosed at 100 and 150 LU/kg flour, the *Fusarium venenatum* lipase yielded a better shape-factor, but lower volume after normal fermentation time than the *Fusarium oxysporum* lipase. After an extended fermentation time, 100 and 150 LU/kg flour of *Fusarium venenatum* lipase performed as well as the *Fusarium oxysporum* lipase.

Firmness and elasticity were followed during the first 24 hours after baking. The *Fusarium venenatum* lipase performed similarly to the *Fusarium oxysporum* lipase on initial softness and elasticity.

The overall optimal dosage of the *Fusarium venenatum* lipase was between 80-150 LU/kg flour, which corresponded to approximately 0.4-0.7 mg enzyme/kg flour. The optimal performance of *Fusarium venenatum* lipase appeared to be very close to that of the *Fusarium oxysporum* lipase at the optimal dosage of 1000 LU per kg flour corresponding to 0.33 mg enzyme/kg flour.

## Claims

1. A polynucleotide which comprises a nucleotide sequence encoding a lipolytic enzyme, which sequence:
a) is a DNA sequence cloned into a plasmid present in *Escherichia coli* deposit number *Escherichia coli* DSM 13539;
b) is the mature polypeptide-encoding DNA sequence shown in SEQ ID NO: 3 or is a DNA sequence that can be derived there from by substitution, deletion, and/or insertion of one or more nucleotides; or
c) has at least 80% identity with the mature polypeptide-encoding sequence of SEQ ID NO: 3; or
d) a complementary strand of the sequence defined in a), b) or c).

2. The polynucleotide of claim 1 which is native to a strain of *Fusarium,* particularly *F. sulphureum.*

3. A polypeptide having lipolytic enzyme activity which is:
a) a polypeptide having an amino acid sequence which has at least 85% identity with the mature polypeptide of SEQ ID NO: 4;
b) a polypeptide which is encoded by a nucleic acid sequence which hybridizes under high stringency conditions with a complementary strand of the nucleic acid sequence encoding the mature polypeptide of SEQ ID NO: 3 or a subsequence thereof having at least 100 nucleotides;
c) a polypeptide having an amino acid sequence which can be obtained from the mature polypeptide of SEQ ID NO: 4 by substitution, deletion, and/or insertion of one or more amino acids; or
d) a polypeptide encoded by the lipolytic enzyme encoding part of the DNA sequence cloned into a plasmid present in *Escherichia coli* deposit number DSM 13539.

4. The lipolytic enzyme of claim 3 which is native to a strain of *Fusarium,* particularly *F. sulphureum.*

5. The lipolytic enzyme of claim 3 or 4 which has an amino acid sequence comprising the mature polypeptide of SEQ ID NO: 4.

6. A polynucleotide comprising a nucleic acid sequence which encodes the lipolytic enzyme of any of claims 3-5.

7. A nucleic acid construct comprising the polynucleotide of claim 1, 2 or 6 operably linked to one or more control sequences capable of directing the expression of the lipolytic enzyme in a suitable expression host.

8. A recombinant expression vector comprising the nucleic acid construct of claim 7, a promoter, and transcriptional and translational stop signals.

9. A recombinant host cell comprising the nucleic acid construct of claim 7.

10. A method for producing a lipolytic enzyme comprising cultivating the host cell of claim 10 under conditions conducive to production of the lipolytic enzyme, and recovering the lipolytic enzyme.

11. A method for producing a mutant nucleic acid sequence, comprising
a) introducing at least one mutation into the mature polypeptide coding sequence of SEQ ID NO: 3; and
b) recovering the mutant nucleic acid sequence.

12. A method for producing a polypeptide comprising
a) introducing at least one mutation into a polynucleotide having the mature polypeptide coding sequence of SEQ ID NO: 3;
b) transforming a host cell with the mutated polynucleotide;
c) cultivating the transformed host cell under conditions conducive for production of polypeptides;
d) screening the polypeptides to select a polypeptide having lipolytic enzyme activity; and
e) producing the selected polypeptide.

13. A method of producing a lipolytic enzyme which comprises:
a) shuffling a first polynucleotide comprising the mature polypeptide coding sequence of SEQ ID NO: 3 and a second polynucleotide encoding a lipolytic enzyme and having at least 50% identity to the first polynucleotide;
b) expressing the shuffled polynucleotides to form recombinant polypeptides;
c) screening the polypeptides to select a polypeptide having lipolytic enzyme activity; and
d) producing the selected polypeptide.

14. A method for preparing a dough or a baked product made from the dough, comprising adding the lipolytic enzyme of any of claims 3-5 to the dough.

15. A dough composition comprising the lipolytic enzyme of any of claims 3-5.

16. A detergent composition comprising a surfactant and the lipolytic enzyme of claim any of claims 3-5.
